# EUROPEAN PATENT SPECIFICATION

(11) **EP 1 753 452 B1**
(45) Date of publication and mention of the grant of the patent: **28.10.2015**
(21) Application number: 05756247.2
(22) Date of filing: 03.06.2005
(51) Int. Cl.: A01N 37/18, A61K 39/00

(54) **A METHOD FOR ALTERING THE CD4/CD8 RATIO AND THE MONONUCLEAR CELLULAR INFILTRATE INTO A TUMOR**
VERFAHREN ZUR VERÄNDERUNG DES CD4/CD8-VERHÄLTNISSES UND DES MONONUKLEÄREN ZELLULÄREN INFILTRATS IN EINEN TUMOR
PROCEDE PERMETTANT DE MODIFIER LE RAPPORT CD4/CD8 ET L'INFILTRAT CELLULAIRE MONONUCLEAIRE D'UNE TUMEUR

(30) Priority: 04.06.2004 US 576601 P
(43) Date of publication of application: 21.02.2007
(73) Proprietor: Cel-Sci Corporation, Vienna, VA 22182 (US)
(72) Inventor: TALOR, Eyal, Baltimore, MD 21209 (US)
(74) Representative: Potter Clarkson LLP
(86) International application number: PCT/US2005/019263
(87) International publication number: WO 2005/120550

(56) References cited:
- WO-A2-2005/007086
- US-A- 5 698 194
- US-A- 6 017 544
- US-A1- 2001 006 681
- FEINMESSER R ET AL: "REPORT OF A CLINICAL TRIAL IN 12 PATIENTS WITH HEAD AND NECK CANCER TREATED INTRATUMORALLY AND PERITUMORALLY WITH MULTIKINE" ARCHIVES OF OTOLARYNGOLOGY HEAD AND NECK SURGERY, AMERICAN MEDICAL ASSOCIATION, US, vol. 129, 1 August 2003 (2003-08-01), pages 874-881, XP008059890 ISSN: 0886-4470
- TIMAR J ET AL: "THE EFFECT OF LEUKOCYTE INTERLEUKIN INJECTION (MULTIKINE) TREATMENT ON THE PERITUMORAL AND INTRATUMORAL SUBPOPULATION OF MONONUCLEAR CELLS AND ON TUMOR EPITHELIA: A POSSIBLE NEW APPROACH TO AUGMENTING SENSITIVITY TO RADIATION THERAPY AND CHEMOTHERAPY IN" LARYNGOSCOPE, TRIOLOGICAL FOUNDATION, ST LOUIS, US, vol. 113, 1 December 2003 (2003-12-01), pages 2206-2217, XP008048121 ISSN: 0023-852X
- CHIRIGOS M A ET AL: "LEUKOCYTE INTERLEUKIN, INJ. (LI) AUGMENTATION OF NATURAL KILLER CELLS AND CYTOLYTIC T-LYMPHOCYTES" IMMUNOPHARMACOLOGY AND IMMUNOTOXICOLOGY, MARCEL DEKKER, NEW YORK, NY, US, vol. 17, no. 2, 1 January 1995 (1995-01-01), pages 247-264, XP008048122 ISSN: 0892-3973
- TÍMÁR JÓZSEF ET AL: "Neoadjuvant immunotherapy of oral squamous cell carcinoma modulates intratumoral CD4/CD8 ratio and tumor microenvironment: a multicenter phase II clinical trial." JOURNAL OF CLINICAL ONCOLOGY : OFFICIAL JOURNAL OF THE AMERICAN SOCIETY OF CLINICAL ONCOLOGY 20 MAY 2005, vol. 23, no. 15, 20 May 2005 (2005-05-20), pages 3421-3432, XP002531443 ISSN: 0732-183X
- GEZ E. ET AL . CANCER INVEST vol. 17, no. 4, 1999, pages 259 - 263, XP008078987
- HERNBERG ET AL. ANN. ONCOL vol. 8, 1997, pages 71 - 77, XP003010747
- CHAN B. ET AL CYTOTHERAPY vol. 5, 2003, pages 46 - 54, XP008078988
- WEI Y.-F. ET AL ZHONGUO ZHONG ZI YI JIE HE ZA ZHI vol. 23, 2003, pages 258 - 260, XP008079058

## Description

The patent or application contains at least one drawing executed in color. Copies of this application or patent application publication with color drawings(s) will be provided by the Office upon request and payment of the necessary fee.

### INTRODUCTION

This invention relates to a method for altering the CD4/CD8 ratio and the mononuclear cellular infiltrate into a tumor. A significant modulation of the infiltrating immune cells is shown by a decrease in CD8+ T cells and a significant increase in tumor infiltrating CD4+ T cells with a 800 IU/day as IL-2 for 5 days per week dose of Leukocyte Interleukin Injection (LI) treatment for a total of 3 weeks. The low CD4/CD8 ratio seen in a control non-treated group of Oral Squamous Cell Carcinoma (OSCC) patients increased dramatically in the stroma and intraepithelially as a result of LI treatment. A dose of 800 IU/day as IL-2 for 5 days per week for 3 weeks of neoadjuvant LI treatment of advanced primary OSCC patients also resulted in a paradigm shift, the paradigm shift being defined as a marked CD4+ T cell infiltrate and a clear and specific change in the density and localization of antigen presenting cells such as dendritic cells and inflammatory cells, particularly neutrophils in cancer nests.

The Leukocyte Interleukin Injection (LI) is a serum-free and mitogen-free mixture comprised of specific ratios of cytokines IL-1β, TNF-α, IFN-γ and GM-CSF to Interleukin 2 (IL-2). The LI is also effective in inducing cancerous cells to enter a proliferative cell cycle phase thereby increasing their vulnerability to chemotherapy, radiation therapy and immuno-therapy. The LI can be used alone or in combination with other drugs for the treatment of cancer thereby increasing the success of cancer treatment and the disease free survival of cancer patients as described in U.S. Patent application no. 10/611,914 filed on July 3, 2003, published as US 2005-0002896 A1.

WO 2005/007086 A2, prior art according to Art. 54(3) EPC, is directed at a method of pre-sensitizing cancer prior to treatment with radiation and/or chemotherapy and a cytokine mixture used in this method.

Feinmesser, R. et al. (2003) Arch. Otolaryngol. Head Neck Surg. 129: 74-881 and Tímár, J. et al. (2003) Laryngoscope 113: 2206-2217 evaluated the effectiveness and toxicity of LI in the treatment of head and neck cancer and its effects on peritumoral and intratumoral subpopulations of mononuclear cells in OSCC. Tímár, J. et al. (2005) J. Clin. Oncol. 23: 3421-3432 investigated the clinicopathologic effects of local neoadjuvant LI regimen in OSCC.

Gez et al. (1999) Cancer Investigation 17: 259-263 investigate T-cell subpopulations in 10 patients with metastatic renal cell carcinoma treated by recombinant interleukin-2, recombinant interferone-α, 5-fluorouracil, and vinblastine.

### BACKGROUND OF THE INVENTION

Tumor-host interaction is a complex feature of tumor progression and is an increasingly important target for anticancer therapy (Tímár et al., "Molecular pathology of tumor metastasis III. Target array and combinatorial therapies" Pathol Oncol Res 9:49(2003)). Cellular interactions between cancer cells, immune effector cells, inflammatory cells, tumor vasculature and the stroma exhibit complex multifaceted interaction. The therapeutic modulation of the function of individual members of this complex interactive network may result in greater tumor control.

Successful immunotherapeutic anti-cancer effect generally results in the expression of tumor- and MHC antigens on the target tumor cells, cancer cell sensitivity to effector mechanisms, and induced/augmented activity of the anticancer effector mechanisms (Whiteside, "Immunobiology and immunotherapy of head and neck cancer", Current Oncol Rep 3:46 (2001); Whiteside et al., "Evidence for local and systemic activation of immune cells by peritumoral injections of interleukin 2 in patients with advanced squamous cell carcinoma of the head and neck", Cancer Res 53:5654 (1993)). There are several feasible immune strategies to achieve these goals through the use of cytokines to augment immune effector mechanisms. Oral squamous cell carcinoma (OSCC) was not considered until recently an immunotherapeutic target but pioneer studies by Whiteside and others suggested that immunotherapeutic approaches could well be a new way to manage this otherwise highly aggressive cancer.

Early results demonstrated highly variable response rates for local rhIL-2 administrations (6-65%) (Vlock et al., "Phase Ib trial of the effect of peritumoral and intranodal injections of interleukin-2 in patients with advanced squamous cell carcinoma of the head and neck: an eastern cooperative oncology group trial", J Immunother 15:134 (1994); Cortesina et al., "Interleukin-2 injected around tumor-draining lymph nodes in head and neck cancer", Head Neck 13:125 (1991)). Phase-III trials involving the local administration of rhIL-2 to OSCC patient resulted in a significant (p<0.03) increase in disease free survival as well as overall survival (De Stefani et al., "Treatment of oral cavity and oropharynx squamous cell carcinoma with perilymphatic interleukin-2: clinical and pathologic correlations", J Immunother 19:125 (1996); De Stefani et al., "Improved survival with perilymphatic interleukin-2 in patients with respectable squamous cell carcinoma of the oral cavity and oropharynx", Cancer 95:90 (2002)). Histological examination of the tumor tissue obtained from rhIL-2 treated OSCC patients demonstrated enrichment in CD25+/HLADR+/CD3+ T cells with focal necrosis (Valente et al., "Infiltrating leukocyte populations and T-lymphocyte subsets in head and neck squamous cell carcinoma from patients receiving perilymphatic injections of recombinant interleukin 2", Modern Pathol 3:702 (1990).

Clinical trials using natural IL-2 as well as a mixture of naturally occurring cytokines provided response rates of 30% to 53.3% (Hadden et al., "Interleukins and contrasuppression induce immune regression of head and neck cancer", Arch Otolaryngol Head Neck Surg 120:395 (1994); Verastegui et al., "A natural cytokine mixture (IRX-2) and interference with immune suppression induce immune mobilization and regression of head and neck cancer", Int J Immunopharmac 19:619 (1997); Meneses et al., "Histologic findings in patients with head and neck squamous cell carcinoma receiving perilymphatic natural cytokine mixture (IRX-2) prior to surgery", Arch Pathol Lab Med 122:447 (1998); Barrera et al., "Combination Immunotherapy of squamous cell carcinoma of the head and neck", Arch. (2000)). Histopathologic analysis indicated that natural cytokine mixture induced predominant dose dependent T lymphocytic infiltration of the tumor and tumor fragmentation.

On the other hand, the serum-free and mitogen-free Leukocyte Interleukin Injection (LI) mixture comprised of specific ratios of cytokines IL-1β, TNF-α, IFN-γ and GM-CSF to Interleukin 2 (IL-2) induced CD3+, CD25+ T lymphocytic infiltration of tumor cell nests as well as entry of the cancer cells into cell cycle mode (Tímár et al., "The Effect of Leukocyte Interleukin Injection (Multikine®) Treatment on Peritumoral and Intratumoral Subpopulation of Mononuclear Cells and on Tumor Epithelia: A Possible New Approach to Augmenting Sensitivity to Radiation Therapy and Chemotherapy in Oral Cancer", Laryngoscope 113:2206 (2003)).

As reported in Tímár et al. Leukocyte Interleukin (LI) Injection was administered peritumorally three times a week over a two week period in a range from about 20 IU to 1600 IU or specifically at 400 IU or at 800 IU or still further at five times a week in a range from about 20 IU to 1600 IU or at 400 IU or at 800 IU wherein IU represent International Units for Interleukin-2 given in World Health Organization 1^{st} International Standard for Human IL-2, 86/504. The doses were administered to advanced primary OSCC patients as a first-line treatment prior to conventional therapy and induced intra-tumor alterations in infiltrating immune cells rather than inflammatory cells. The T cell population migrated into the cancer nests and the infiltrating CD8+ T cells were activated as demonstrated by increased CD25+ expression.

Although it is generally accepted in the scientific literature that CD8+ cytotoxic T cells are required for tumor destruction, the lack of HLA class I expression on neoplastic cells in OSCC appears to preclude CD8+ cell targeting in OSCC. Therefore, a successful immune response to OSCC may require the reversal of a low intratumoral CD4/CD8 ratio.

Notably, the predominance of CD8+ T cells over CD4+ T cells in the naïve non-treated OSCC patient within the tumor and the surrounding stroma is not specific for OSCC and can be seen in many other solid tumors. Similarly low CD4/CD8 ratios have been detected in basal cell carcinoma, cervical carcinoma and in breast cancer suggesting an acquired immuno-suppressed state of the patient induced by these tumors (Rohrbach et al., "Immunology and growth characteristics of ocular basal cell carcinoma", Graefes Arch Clin Exp Ophthalmol 239:35 (2001); Santin et al., "Tumor-infiltrating lymphocytes contain higher numbers of 1 cytokine expressors and DR+ T cells compared with lymphocytes from tumor draining lymph nodes and peripheral blood in patients with cancer of the uterine cervix", Gynecol Oncol 81:424 (2001); Ben-Hur et al., "The role of lymphocytes and macrophages in human breast tumorigenesis: an immunohistochemical and morphometric study", Anticancer Res 22(2B):1231 (2002)).

In combination, these studies and observations demonstrate that local treatment with a mixture of natural cytokines is a feasible approach to the treatment of OSCC. However, the relationship between the induced alterations in the cellular infiltrate of the tumor, changes in tumor histology and the clinically determined responses was not evaluated.

Therefore, there is a need for specific modulation in the density and localization of antigen presenting cells (dendritic cells) and inflammatory cells, especially neutrophils, in the cancer nests. There is also a need to shift the makeup of the tumor immune cell infiltrate to reverse the intra-tumoral CD4/CD8 ratio. There is also a need to provide for a method for altering the CD4/CD8 ratio and the mononuclear cellular infiltrate into a tumor in conjunction with administering LI to increase sensitization to radiation therapy of the residual tumor cells.

There is still also a need for method for inducing tumor cells into a cell cycle selected from the group of (different phases of the cell cycle) G₁, S, G₂ and M where the new methods can be synergistically applied with chemotherapy, immuno-therapy and radiation therapy and by altering the CD4/CD8 ratio and the mononuclear cellular infiltrate into a tumor.

There is also a need for pre-sensitizing cancer tumors in general along with the need for a new serum-free and mitogen-free cytokine mixture comprised of specific ratios of IL-1β to IL-2, TNF-α to IL-2, IFN-γ to IL-2 and GM-CSF to IL-2 that unexpectedly demonstrates efficacy over known compositions or methods by altering the CD4/CD8 ratio and the mononuclear cellular infiltrate into a tumor.

### SUMMARY OF THE INVENTION

The present invention is based, in part, on methods of altering the CD4/CD8 ratio and the mononuclear cellular infiltrate into a tumor with a serum-free and mitogen-free Leukocyte Interleukin Injection (LI) mixture comprised of specific ratios of cytokines IL-1β to IL-2, TNF-α to IL-2, IFN-γ to IL-2 and GM-CSF to IL-2. The present invention also enables the development of compositions useful as a pharmaceutical or as an adjuvant to be used in conjunction with therapeutic cancer treatments such as chemotherapy, immuno-therapy and radiation therapy.

In embodiments of the disclosure, a method is disclosed for altering the CD4/CD8 ratio in a neoplasm or disease of the immune system with a serum-free and mitogen-free cytokine mixture. The methods provide for a pre-sensitizing step for the treatment of cancer in conjunction with radiotherapies or other physical modalities of tumor cell killing. A method for inducing tumor cells into a vulnerable cell cycle phase selected from the group of (different phases of the cell cycle) G1, S, G₂ and M is also contemplated.

Specific applications include administering half a total daily dose peritumorally of a Leukocyte Interleukin, Injection (LI) and administering the other half of the total daily dose perilymphatically five (5) times per week for three (3) weeks. The total daily dose is 800 IU/mL as IL-2 wherein IU represents International Units for Interleukin-2 given in World Health Organization 1 International Standard for Human IL-2, 86/504. Half the total dose is the peritumoral dose. Therefore, 400 IU is injected peritumorally into four different sites around the tumor mass. One quarter (1/4) of the peritumoral dose is injected into each of the four sites such that each site receives approximately 100 IU/mL as IL-2. The LI is administered intradermally at the circumferential margins of the visible/palpable tumor mass. The remaining half of the total dose of 400 IV as IL-2 is sequentially administered perilymphatically ipsilateral to the tumor site on the same visit. The perilymphatic injections are administered at the posterior mandibular area in the area of the jugular lymphatic chain ipsilateral to the injected tumor mass,

the Leukocyte Interleukin, Injection (LI) having specific ratios of cytokine to interleukin 2 (IL-2) as follows: IL- 1β to IL-2 at a ratio range of 0.4 - 1.5, and preferably at 0.7+/- 0.1 (IL-1β/IL-2), TNF- α to IL-2 at a ratio range of 3.2 - 10.9, and preferably at 9.5+/- 1.8 (TNF-α/IL-2), IFN-γ to IL-2 at a ratio range of 1.5 - 10.9, and preferably at 6.0+/- 1.1 (IFN-γ/IL-2), and GM-CSF to IL-2 at a ratio range of 2.2 - 4.8, and preferably at 4.0+/- 0.5 (GM-CSF/IL-2).

In other specific applications described herein, the serum-free and mitogen-free cytokine preparation or pharmaceutical composition has further different cytokines and other small biologically active molecules wherein the ratio of each of the small biologically active molecules to IL-2 is as follows: IL-3 to IL-2 in a ratio range of 0.38 - 0.68, preferably at 0.53+/- 0.15, IL-6 to IL-2 in a ratio range of 37.2 - 53.8, preferably at 46+/- 5.9, IL-8 to IL-2 in a ratio range of 261 - 561.5, preferably at 411+/- 10.6, IL-1 α to IL-2 in a ratio range of 0.56 - 0.94, preferably at 0.75+/- 0.19, IL-10 to IL-2 in a ratio range of 2.82 - 3.22, preferably at 3.0+/- 0.18, IL-16 to IL-2 in a ratio range of 1.16 - 2.84, preferably at 1.84+/-0.68, G-CSF to IL-2 in a ratio range of 2.16 - 3.78, preferably at 2.97+/- 0.81, TNF-β to IL-2 in a ratio range of 1.17 - 2.43, preferably at 1.8+/- 0.63, M1P-1α to IL-2 in a ratio range of 15.7 - 37.16, preferably at 22.7+/-7.0, MIP-1β to IL-2 in a ratio range of 17.1 - 28.5, preferably at 22.8+/- 5.7, a RANTES to IL-2 in a ratio range of 2.3 - 2.7, preferably at 2.5+/- 0.13, a EGF to IL-2 in a ratio range of 0.267 - 0.283, preferably at 0.275+/- 0.008, PGE₂ to IL-2 in a ratio range of 3.63 - 5.42, preferably at 4.5+/- 0.87 and TxB₂ to IL-2 in a ratio range of 23.47 - 25.13, preferably at 24.3+/- 0.83.

Other objects and advantages of the present invention are set forth in the following description. The accompanying drawings and tables, which constitute a part of the disclosure, illustrate and, together with the description, explain the principle of the invention. One of ordinary skill in the art will appreciate that other aspects of this invention will become apparent upon reference to the attached figures and the following detailed description.

### BRIEF DESCRIPTION OF THE DRAWINGS

The invention will now be explained in greater detail by the following description and specific embodiments and with the aid of the accompanying drawings:

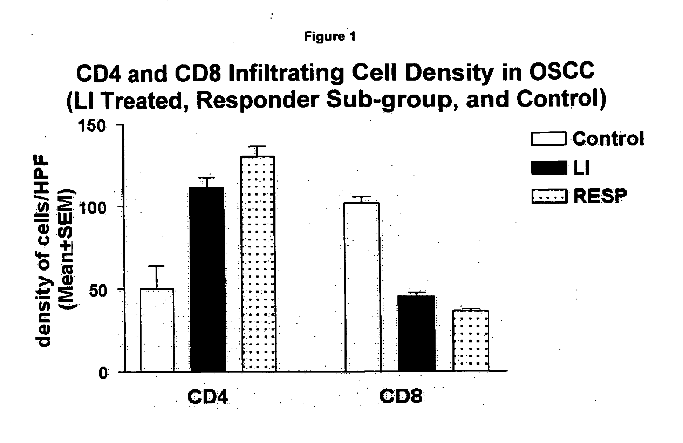

Fig. 1 represents the CD4 and CD8 Infiltrating Cell density of the number of cells per HPF (High Power Field), in LI-treated, Responder (to treatment) sub-group, and control, expressed as Mean ± SEM (increase in the density of CD4+ T cells as compared to control p<0.05, and a decrease in CD8+ T cells as compared to control p<0.05).

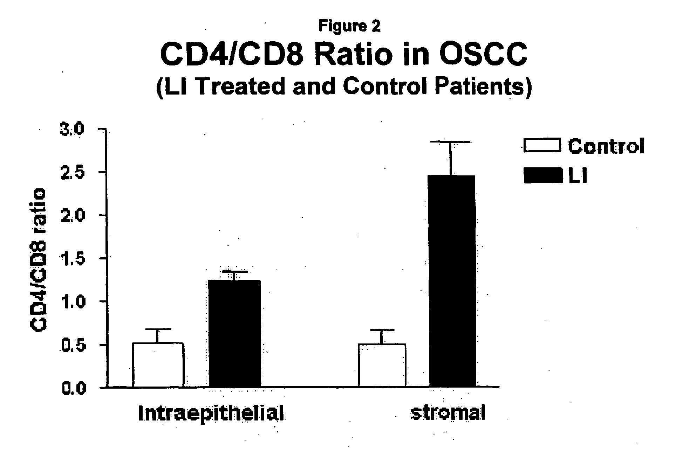

Fig. 2 represents the CD4/CD8 ratio in LI-treated and control, as detected in the OSCC (Oral Squamous Cell Carcinoma) tumor (intraepithelial and stroma). A reversal of the CD4/CD8 ratio as compared to control (CD4/CD8 ratio of <1, in control to CD4/CD8 ratio of >2.5 in LI-treated).

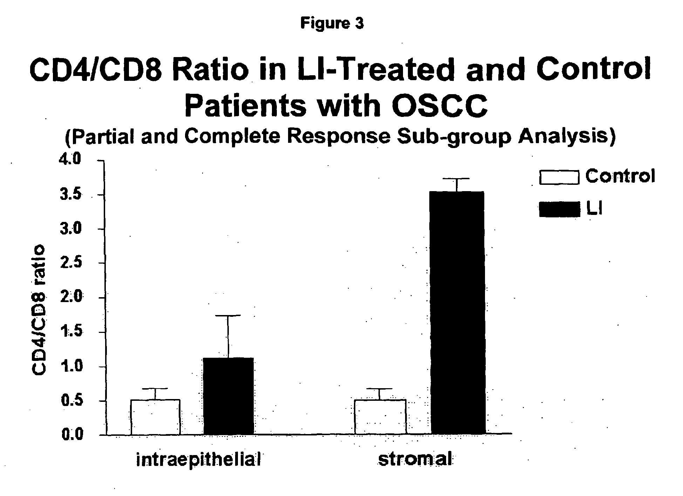

Fig. 3 represents CD4/CD8 ratio in LI-treated (responder sub-group) and control, as detected in the OSCC (Oral Squamous Cell Carcinoma) tumor (intraepithelial and stroma). A reversal of the CD4/CD8 ratio as compared to control (CD4/CD8 ratio of <1, in control to CD4/CD8 ratio of >3.5 in LI-treated responder sub-group).

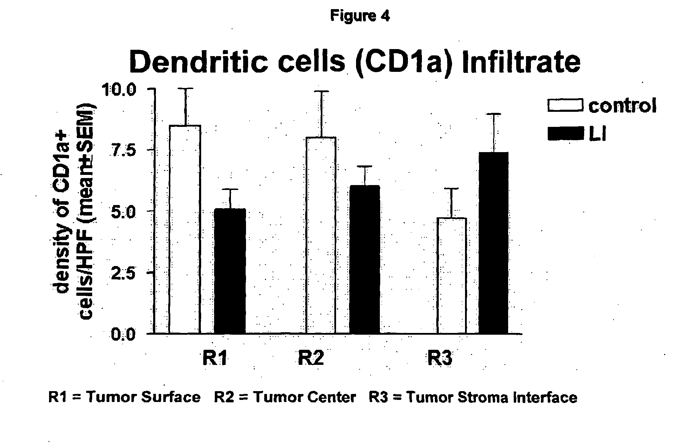

Fig. 4 represents dendritic (CD1a) cells infiltration and localization in the OSCC (Oral Squamous Cell Carcinoma), in the tumor surface (R1), tumor center (R2) and tumor stroma interface (R3). Number of CD1a+ cells in HPF (High Power Field), expressed as Mean ± SEM, in LI-treated and control.

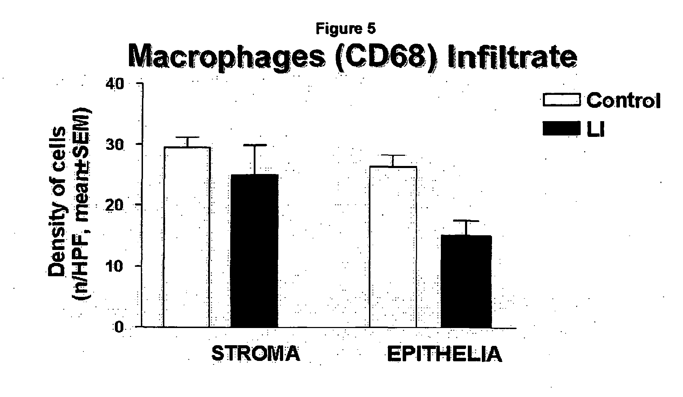

Fig. 5 represents macrophages (CD68) cells infiltration in the OSCC (Oral Squamous Cell Carcinoma), in the tumor stroma and tumor epithelia. Number of CD68+ cells in HPF (High Power Field), expressed as Mean ± SEM, in LI-treated and control (a decrease in the number of macrophages in LI-treated as compared to control, intraepithelially, p<0.002).

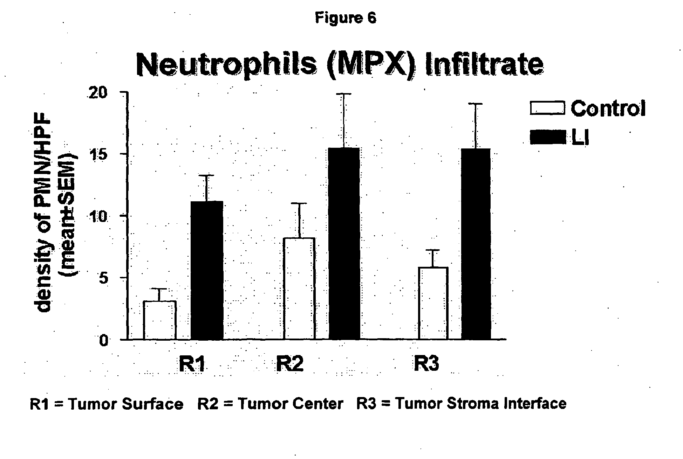

Fig. 6 represents neutrophils (MPX, Myeloperoxidase) cells infiltration and localization in the OSCC (Oral Squamous Cell Carcinoma), in the tumor surface (R1), tumor center (R2) and tumor stroma interface (R3). Number of MPX+ cells in HPF (High Power Field), expressed as Mean ± SEM, in LI-treated and control (an increase in the intraepithelial density of neutrophils in the LI-treated group as compared to control, p<0.005).

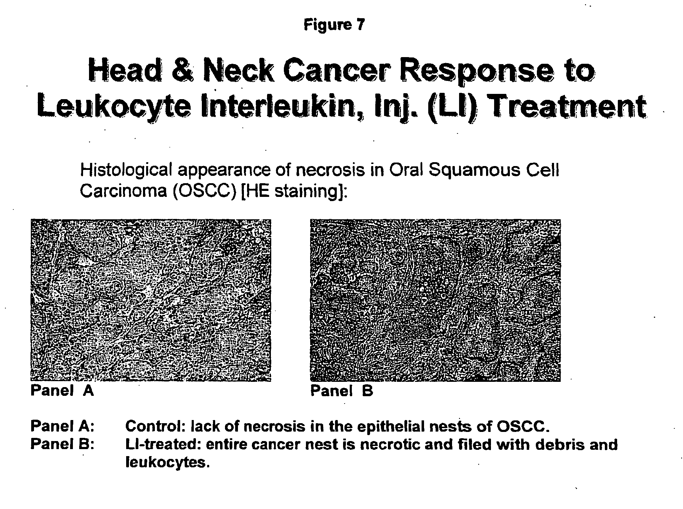

Fig. 7 represents histological appearance of necrosis in OSCC (H&E staining). Panel "A" - control showing lack of necrosis in the intraepithelial nest of OSCC, and Panel "B" - LI-treated showing the entire cancer nest to be necrotic, filled with debris and leukocytes.

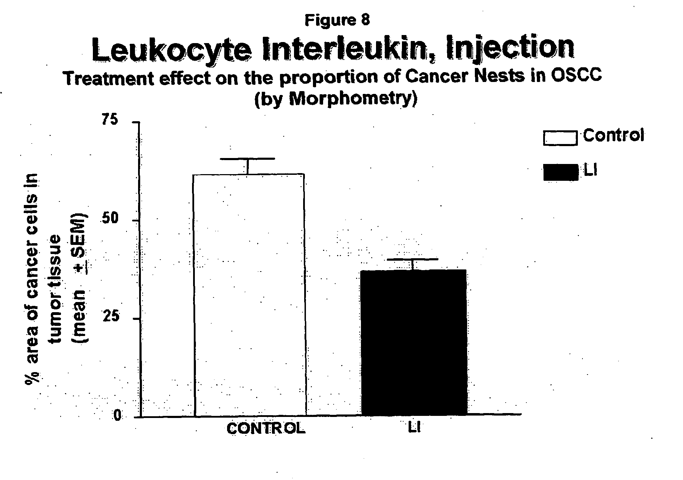

Fig. 8 represents treatment effect on the proportion of cancer nests in OSCC (Oral Squamous Cell Carcinoma) in LI-treated and control group (as determined by morphometric analysis). A decrease in the proportion of cancer nests in the LI-treated group as compared to control, p<0.005).

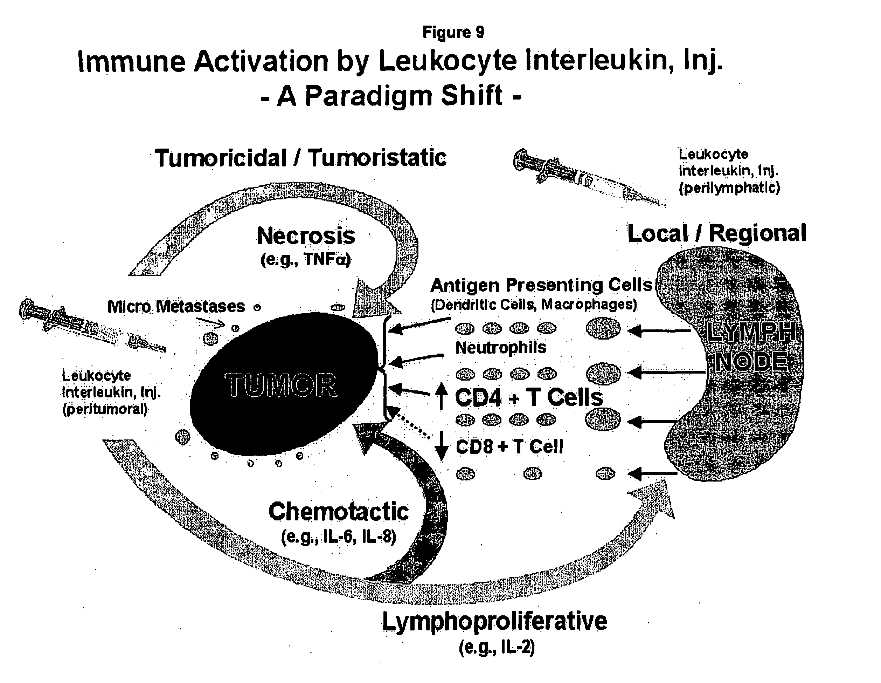

Fig. 9 represents immune activation by Leukocyte Interleukin, Injection (LI). LI contains cytokines, chemokines, and Lymphoproliferative lymphokines working in concert to bring about a coordinated immune attack on the tumor *in vivo.* LI is injected in the vicinity if the tumor and local lymph nodes. First, necrotic factors such as TNF-α cause tumor cell death releasing tumor antigens. Second, antigen presenting cells such as dendritic cells at the tumor-stroma interface process and present these tumor antigens to CD4+ T cells. Third, Lymphoproliferative lymphokines such as IL-2 cause T cell proliferation both at the local lymph node and at the tumor site. Fourth, Chemotactic factors in LI such as IL-6, IL-8, and others guide tumor specific immune cells and inflammatory cells to the tumor site to affect immune cell infiltration into the tumor and cause inflammatory response to the tumor culminating in tumor destruction.

### DETAILED DESCRIPTION OF THE INVENTION AND PREFERRED EMBODIMENTS

The scope of the present invention is defined by the claims and any information that does not fall within the claims is provided for information only.

The present invention is concerned with methods of pre-sensitizing cancer in general and a novel serum-free and mitogen-free cytokine mixture comprised of specific ratios of IL-1β to IL-2, TNF-α to IL-2, IFN-γ to IL-2 and GM-CSF to IL-2. One such novel cytokine mixture is Leukocyte-Interleukin Injection (LI), which has demonstrated immuno-modulatory capabilities. The clinical significance of the immuno-suppression in cancer patients unexpectedly impacts methods of pre-sensitizing cancer prior to therapeutic treatment and in particular entry of the tumor cell into a cell cycle phase.

Moreover, administering 400 IU/mL peritumorally of a Leukocyte Interleukin, Injection (LI) and administering 400 IU/mL perilymphatically five (5) times per week for three (3) weeks altered the CD4/CD8 ratio and the mononuclear cellular infiltrate into a tumor. The peritumoral dose is performed by injecting 100 IU/mL as IL-2 into four different sites around the tumor mass. Each of the four sites is injected with one quarter (1/4) of the entire 400 IU/mL peritumoral dose such that each site receives approximately 100 IU/mL as IL-2. The remaining half of the total dose of 800 IU as IL-2 is administered perilymphatically ipsilateral to the tumor site sequentially and on the same visit. The perilymphatic injections are given at one site at the posterior mandibular area in the area of the jugular lymphatic chain ipsilateral to the injected tumor mass. IU represents International Units for Interleukin-2 given in World Health Organization 1^{st} International Standard for Human IL-2, 86/504.

Leukocyte-Interleukin Injection (LI) is a serum-free, mitogen-free, antibiotic-free preparation produced from human peripheral blood mononuclear cells that include T-cells, B cells and macrophages. There are three "families" of cytokines in LI that together are important to the unique biological activity of LI. They include direct cytotoxic/cytostatic and virocidal/virostatic cytokines such as TNF-α, and IFN-γ, lympho-proliferative cytokines such as IL-1, and IL-2 and chemotactic cytokines such as IL-6, IL-8 and MIP-1α. Furthermore, the different cytokine and small biological molecules that constitute LI are all derived from the lectin (PHA) *in vitro* stimulation of human peripheral blood mononuclear cells that include T cells, B cells, and macrophages. Centrifugation on a Ficoll-Paque gradient separates the white blood cells (including T cells, B cells, and macrophages) from donor whole blood, and a series of washes (in physiologically buffered media) facilitates the isolation of lymphocytes, and the removal of red blood cells, cellular debris and other unwanted cellular components from the isolated white cell component of the whole donor blood.

LI contains different cytokines present at specific ratios of each cytokine to Interleukin 2 (IL-2) as follows: IL-1β to IL-2 at a ratio range of 0.4 - 1.5, and preferably at 0.7+/- 0.1 (IL-1β /IL-2), TNF-α to IL-2 at a ratio range of 3.2 - 10.9, and preferably at 9.5+/- 1.8 (TNF- α /IL-2), IFN-γ to IL-2 at a ratio range of 1.5 - 10.9, and preferably at 6.0+/- 1.1 (IFN-γ /IL-2), and GM-CSF to IL-2 at a ratio range of 2.2 - 4.8, and preferably at 4.0+/- 0.5 (GM-CSF/IL-2).

Also described herein is that the remainder of the different cytokines and other small biologically active molecules in LI are also present within each preparation of the small biologically active molecule to IL-2 is as follows: IL-3 to IL-2 in a ratio range of 0.38 - 0.68, preferably at 0.53+/- 0.15, IL-6 to IL-2 in a ratio range of 37.2 - 53.8, preferably at 46+/- 5.9, IL-8 to IL-2 in a ratio range of 261 - 561.5, preferably at 411+/- 10.6, IL-1α to IL-2 in a ratio range of 0.56 - 0.94, preferably at 0.75+/- 0.19, IL-10 to IL-2 in a ratio range of 2.82 - 3.22, preferably at 3.0+/- 0.18, IL-16 to IL-2 in a ratio range of 1.16- 2.84, preferably at 1.84+/-0.68, G-CSF to IL-2 in a ratio range of 2.16 -3.78, preferably at 2.97+/- 0.81, TNF-β to IL-2 in a ratio range of 1.17 - 2.43, preferably at 1.8+/- 0.63, MIP-1α to IL-2 in a ratio range of 15.7 - 37.16, preferably at 22.7+/- 7.0, MIP-1β to IL-2 in a ratio range of 17.1 - 28.5, preferably at 22.8+/- 5.7, a RANTES to IL-2 in a ratio range of 2.3 - 2.7, preferably at 2.5+/- 0.13, a EGF to IL-2 in a ratio range of 0.267 - 0.283, preferably at 0.275+/- 0.008, PGE₂ to IL-2 in a ratio range of 3.63 - 5.42, preferably at 4.5+/- 0.87 and TxB₂ to IL-2 in a ratio range of 23.47 - 25.13, preferably at 24.3+/- 0.83.

Leukocyte-Interleukin Injection (LI) was tested using a characterization protocol and does not contain the following cytokines and other small biologically active molecules: IL-4, IL-7, and IL-15, TfR, sICAM, PDGF-AB, IFN-α, EPO, LTC 4, TGF-β2, FGF basic, Angiogenin, sE-selectin, SCF, and LIF. LI contains only trace quantities (just above the level of detection of the assay) of IL-12, and LTB 4.

In the manufacturing process, mononuclear cells are separated from human donor "buffy coats" by step-gradient centrifugation and cultured with PHA to enhance production and secretion of IL-2 and other cytokines from the donor white blood cells in culture as disclosed in U.S. Patents 5,093,479, 4,390,623, 4,388,309, 4,406,830, 4,661,447, 4,681,844 and 4,464,355. Subsequently, the culture supernatant is aseptically harvested, clarified and subjected to a commercial virus exclusion process. The supernatant is then further concentrated approximately 10 fold by ultrafiltration and microfiltration.

At this point, Human Serum Albumin, Inj. USP is added and the concentrate is then buffered to a physiological pH and brought to a target IL-2 concentration per the label claim (example 400 IU/mL). The concentrate is then subjected to a second micro-filtration (0.22 micron-rated filter) and aseptically dispensed into sterile serum-type vials and labeled by its IL-2 content. Product potency is measured by the incorporation of radio-labeled thymidine by a cytotoxic T-lymphoid line (CTLL-2). The final injectable agent is further tested by ELISA for the presence of five marker cytokines: IL-2, IL-1β, GM-CSF, IFN-γ, and TNF-α.

### Definitions

IL-2 - Interleukin 2 (IL-2): A 15.5-kD glycoprotein synthesized by CD4+ helper T lymphocytes (Formally known as T cell Growth Factor). IL-2 has an autocrine effect acting on the CD4+ T lymphocytes that produce it and on other cells of the immune system (including B lymphocytes, CD8+ T lymphocytes, NK [Natural Killer] cells and others).

IL-1β - Interleukin 1 beta (IL-1β): A 17-kD cytokine synthesized by activated mononuclear phagocytes is found in free form in the circulation and mediates inflammatory responses. It acts on CD4+ T lymphocytes to help facilitate their proliferation, and acts on B-lymphocytes as a growth and differentiation factor. It also induces the synthesis of IL-6 by mononuclear phagocytes.

TNF-α - Tumor Necrosis Factor alpha (TNF-α): A 157 amino acid (aa) residues protein, synthesized by stimulated monocytes, macrophages, B lymphocytes, T lymphocytes, an NK cells among others, found in a trimmeric form in the circulation. TNF mediates direct anti-tumor action, causing tumor cell lysis, facilitates leukocyte recruitment, inducing angiogenesis and promotes fibroblast proliferation.

IFN-γ- Interferon Gamma (IFN-γ): A 21-24-kD glycoprotein homodimer synthesized by activated T lymphocytes and NK cells, is a powerful activator of monocytes increasing monocytes ability to destroy intracellular microorganisms and tumor cells. It has direct anti-viral and anti-proliferative activity, and causes many cell types to express Class II MHC (Major Histocompatibility Complex) cell surface molecular complex, as well as increasing the expression of Class I MHC.

GM-CSF - Granulocyte Macrophage - Colony Stimulating Factor (GM-CSF): A 127 aa protein found as a monomer in the circulation, produced by macrophages and T lymphocytes, fibroblast and endothelial cells. It is a growth factor for hemopoietic cells, and stimulates the growth and differentiation of myelomonocytic lineage.

IL-3 - Interleukin - 3 (IL-3): A 20-kD lymphokine synthesized by activated CD4+ T helper lymphocytes, acts as a colony-stimulating factor by facilitating the proliferation of some hematopoietic cells and promoting the proliferation and differentiation of T lymphocytes.

IL-6 - Interleukin - 6 (IL-6): A 26-kD cytokine produced by activated T lymphocytes, mononuclear phagocytes, endothelial cells, and fibroblasts. It acts on many cells but has a special function in enabling activated B-lymphocytes to differentiate into antibody secreting plasma cells, and induces hepatocytes to form acute-phase proteins (implicated in inflammatory responses) as well as fibrinogen.

IL-8 - Interleukin - 8 (IL-8): An 8-kD protein produced by macrophages and endothelial cells. Is a powerful chemotactic factor for neutrophils and T lymphocytes, and facilitates neutrophil adherence to endothelial cells.

IL-1α - Interleukin 1 alpha (IL-1α): A 17-kD cytokine (like IL-1β) is cleaved from a 33-kD precursor molecule, synthesized by activated mononuclear phagocytes, is rarely found in free form in the circulation and acts as a membrane-associated substance. It assists IL-1β in mediating inflammatory responses.

IL-10 - Interleukin - 10 (IL-10): An 18-kD polypeptide produced by CD4+ and CD 8+ T lymphocytes, monocytes, macrophages, activated B lymphocytes, and keratinocytes. It inhibits macrophages ability to present antigen particularly to T_{H}1-type cells, and secrete IL-6 and TNF.

IL-16 - Interleukin - 16 (IL-16): A 14-kD tetrameric protein produced by CD8+ T lymphocytes, eosinophils, mast cells and respiratory epithelial cells. It has strong chemoattraction properties for CD4+ T lymphocytes and monocytes.

G-CSF - Granulocyte Colony Stimulating Factor (G-CSF): A 22 - 25-kD homodimer glycoprotein produced by macrophages, endothelial cells, fibroblasts and stromal cells. It increases granulocyte progenitor cells in the marrow, and sustains increase in blood neutrophils. It also enhances the ability of neutrophils to exhibit enhanced super-oxide production thought to be important in the destruction of microbially infected cells and tumor cells.

TNF-β - Tumor Necrosis Factor beta (TNF-β): A 25-kD protein produced by activated lymphocytes. It can kill tumor cells in culture, and stimulates proliferation of fibroblasts. In addition it mimics most of the other actions of TNF-α.

MIP-1α - Macrophage Inflammatory Protein - 1 alpha (MIP-1α): A 66-aa monomeric protein produced by macrophages and other cells. It is a chemo-attractant for monocytes, T lymphocytes and eosinophils.

RANTES - An 8-kD protein produced by T lymphocytes and is a chemo-attractant to monocytes, T lymphocytes and eosinophils, and promotes inflammation.

EGF - Epidermal Growth Factor (EGF): A trisulfated polypeptide of 53-aa residues. EGF is a member of the tyrosin kinase family, and has multiple functions including stimulation of the mitogenic response and assisting in wound healing.

PGE₂ - Prostaglandin E₂ (PGE₂): PGE₂ belong to a family of biologically active lipids derived from arachidonic acid through the cyclooxygenase enzymatic reaction. It is released by activated monocytes and blocks MHC Class II expression on T lymphocytes and macrophages.

TxB₂ - Thromboxane B₂ (TxB₂): TxB₂ is a member of biologically active compounds derived from polyunsaturated fatty acids by isomerization of prostaglandin and endoperoxidase PGH₂ via the enzyme thromboxane synthetase. TxB₂ has a physiological role in thromboembolic disease, and anaphylactic reactions.

CD25⁺ Cells - CD25 is a single chain glycoprotein, often referred to as the α-chain of the Interleukin-2-receptor (IL-2R) or the Tac-antigen, that has a mol wt of 55 kDa and is present on activated T and B cells and activated macrophages. It functions as a receptor for IL2. Together with the β-chain of the IL-2R, the CD25 antigen forms a high-affinity receptor complex for IL-2.

CTLL-2 (Cell Line) - A line of mouse cytotoxic T lymphocytes obtained from C57B1/6 mice. This T cell line is dependent on an exogenous source of IL-2 for growth and proliferation.

Fas - FasL-The Fas/Fas Ligand system. The combination of a Fas antigen, a cell surface transmembrane protein that mediates apoptosis, and a complementary Fas-activated cytokine on a neutrophil that transduces an apoptotic signal into cells. Fas is a type-I membrane protein belonging to the tumor necrosis factor (TNF) receptor superfamily, and FasL is a member of the TNF family. FAS ligand is a membrane-bound protein of 31 kDa [kilo Dalton] (278 amino acids). The Fas-Fas ligand system plays important roles in many biological processes, including the elimination of autoreactive lymphoid cells. The Fas ligand is predominantly expressed in activated T lymphocytes and is one of the major effector molecules of cytotoxic T lymphocytes and natural killer cells.

HLA-DR⁺ Lymphocytes - Lymphocytes containing human leukocyte antigen (HLA)-DR antigens, a group of polymorphic glycoproteins determined by a glue sequence found in a leukocyte loci located on chromosome 6, the major histocompatibility loci in humans.

IU (International Units) - A unit of measure of the potency of biological preparations by comparison to an international reference standard of a specific weight and strength e.g., WHO 1^{st} International Standard for Human IL-2, 86/504. International Units are the only recognized and standardized method to report biological activity units that are published and are derived from an international collaborative research effort.

U (Units as a measure of biological activity) - Shorthand for a variety of named "units", which each laboratory derives as a reference, which is further unique to the laboratory where the work is being performed. Each "unit" is different from one laboratory to another laboratory and is not a globally recognized standard such as International Units (IU) .

Mononuclear Infiltrate - Presence of monocytes, plasma cells, and lymphocytes, in tissue where they "normally" would not be present; or the presence of these cells in large numbers or abundance in clusters where they would otherwise be present in only a small number.

TCR ζ Chain - T-cell receptor-zeta chain. The zeta subunit is part of the TCR complex and is targeted towards the interaction of the TCR cell surface receptor with its ligand (antigen). The zeta subunit extending into the cell cytoplasm (cytosol) is phosphorylated at its tyrosine residues upon T cell activation and is implicated in signal transduction after TCR ligation.

TIL (Tumor Infiltrating Lymphocytes) - T lymphocytes isolated from the tumor they are infiltrating. Tumor Infiltrating lymphocytes have little or no cytotoxicity. TILs include CD4+ CD8+ predominantly T cells, and can be expanded *in vitro* by culture in the presence of IL-2. These cells are activated by the treatment with IL-2 and are frequently more aggressive towards the tumor from which they were isolated than normal lymphokine activated cells. The cytotoxic activity of TILs can be enhanced by IFN-γ. The antitumor activity of TILs *in vivo* can be blocked by TGF-β.

ZAP 70 - A 70 kD Zeta Associated Protein associated with the TCR ζ Chain that is a tyrosine kinase present in cytosol. ZAP 70 is thought to participate in maintaining T lymphocyte receptor signaling, mediating the signal transduction which eventually produces IL-2. The ZAP70 gene is expressed in T-cells and natural killer cells and maps to human chromosome 2q12.

ζ (Zeta) Chain - See TCR ζ Chain- The zeta chain gene is located on chromosome 1 in humans. The extracellular domain of this protein is nine amino acids long whereas the transmembrane domain contains a negatively charged aspartic acid residue and the cytoplasmic domain is 113 amino acids long. The cytoplasmic tail contains three of the antigen recognition motifs found in the cytoplasmic tails of CD3 chains. The zeta chain is also associated with other receptors such as the Fc (fragment, crystalline)-gamma receptor of NK cells.

USP - U.S. Pharmacopeia Monographs.

*P -* "p < 0.01": A term in mathematical statistics that denotes the level of probability of an event occurring under pre-set conditions.

ANOVA (Analysis of Variances) - A single factor analysis as described in Statistics and mathematical textbooks e.g., "Handbook of Statistical Methods for Engineers and Scientists", Harrison M. Wadsworth, Jr., Ed., McGraw Hill 1990, and "Statistical Operations Analysis of Health Research Data", Robert P. Hirsch and Richard K. Riegelman, Eds. Blackwell Science Inc., 1996.

### Mode of action and characterization of LI

Leukocyte Interleukin Injection (LI) is a biologically active, minimally toxic, immunomodulatory mixture of naturally derived and naturally occurring human cytokines produced under set conditions as described herein. LI can be used as an anti-cancer and anti-viral therapy or as a neo-adjuvant therapy with a broad-spectrum application for cancer, infectious disease, and other diseases states responding to immunomodulation.

Animal studies demonstrate that "mixed interleukins" have immunomodulatory and immunostimulatory activity *in vitro* (Hadden et al., "Mixed Interleukins and Thymosin Fraction V Synergistically Induce T Lymphocyte Development in Hydrocortisone-Treated Aged Mice", Cell. Immunol. 144:228-236 (1992)). Without being limited to any one theory, it is hypothesized that the local/regional injection of "mixed interleukins" overcomes local immuno-suppression. Subsequently, a break tolerance to tumor antigens occurs and allows for an effective local anti-tumor immune response to occur. It has also been known that the local instillation of interleukins in the region of the tumor or the actual transfection of Interleukin genes into a tumor markedly augments the anti-tumor immune response resulting in tumor regression as reported by Golumbek et al., "Treatment of Established Renal Cancer by Tumor Cells Engineered to Secrete Interleukin-4", Science 254:713-716 (1991).

However, inducing malignant cells into a cell cycle phase without causing the active proliferation of the tumor or the effect of altering the CD4/CD8 ratio and the mononuclear cell infiltrate into a tumor of LI was not previously known for compositions having specific ratios of cytokine to interleukin 2 (IL-2) as follows: IL-1β to IL-2 at a ratio range of 0.4 - 1.5, and preferably at 0.7+/- 0.1 (1L-1β/IL-2), TNF-α to IL-2 at a ratio range of 3.2 - 10.9, and preferably at 9.5+/- 1.8 (TNF-α/IL-2), IFN-γ to IL-2 at a ratio range of 1.5 - 10.9, and preferably at 6.0+/- 1.1 (IFN-γ/IL-2), and GM-CSF to IL-2 at a ratio range of 2.2 - 4.8, and preferably at 4.0+/- 0.5 (GM-CSF/IL-2) and further having different cytokines and other small biologically active molecules wherein the ratio of each of the small biologically active molecules to IL-2 is as follows: IL-3 to IL-2 in a ratio range of 0.38 - 0.68, preferably at 0.53+/- 0.15, IL-6 to IL-2 in a ratio range of 37.2 - 53.8, preferably at 46+/- 5.9, IL-8 to IL-2 in a ratio range of 261 - 561.5, preferably at 411+/- 10.6, IL-1α to IL-2 in a ratio range of 0.56 - 0.94, preferably at 0.75+/- 0.19, IL-10 to IL-2 in a ratio range of 2.82 - 3.22, preferably at 3.0+/- 0.18, IL-16 to IL-2 in a ratio range of 1.16 - 2.84, preferably at 1.84+/-0.68, G-CSF to IL-2 in a ratio range of 2.16 - 3.78, preferably at 2.97+/- 0.81, TNF-β to IL-2 in a ratio range of 1.17 - 2.43, preferably at 1.8+/- 0.63, MIP-1α to IL-2 in a ratio range of 15.7 - 37.16, preferably at 22.7+/- 7.0, MIP-1β to IL-2 in a ratio range of 17.1 - 28.5, preferably at 22.8+/- 5.7, a RANTES to IL-2 in a ratio range of 2.3 - 2.7, preferably at 2.5+/- 0.13, a EGF to IL-2 in a ratio range of 0.267 - 0.283, preferably at 0.275+/- 0.008, PGE₂ to IL-2 in a ratio range of 3.63 - 5.42, preferably at 4.5+/- 0.87 and TxB₂ to IL-2 in a ratio range of 23.47 - 25.13, preferably at 24.3+/- 0.83 such as Leukocyte Interleukin, Injection (LI).

Administration of LI pre-surgery leads to an increase in the number of tumor cells in the cell cycle phase without increasing the risk of a more rapidly growing and more rapidly recurring tumor as would otherwise be predicted from the art. LI also alters the CD4/CD8 ratio and the mononuclear cell infiltrate into a tumor. The ability to induce tumor cells into cell-cycle is unique to LI and may be due to the synergistic effect of the different cytokines present in this investigational drug and the differential effect of these cytokines on both the host's immune system and the tumor cells. A paradigm shift is also seen from dose dependent administration of LI altering the CD4/CD8 ratio and the mononuclear cellular infiltrate into a tumor.

### Patients

Information regarding the Leukocyte Interleukin, Injection (LI) treated and control patient-groups are provided in Tables 1 and 2. The primary site of the tumor for both the treated and control groups are presented in Tables 1 and 2, respectively. Forty-one patients are included but only thirty nine are evaluable. These control specimens are matched to a treated group based on tumor size, site of tumor, tumor stage, sex, and age of the patient.

**Table 1**

| Leukocyte Interleukin, Injection Treated Group: Patient Information | | | |
|---|---|---|---|
| Patient # | Sex | Age (year) | Tumor localization |
| *1* | female | 65 | Radix linguae |
| 2 | male | 40 | Tongue |
| 3 | male | 51 | Gingival |
| 4 | male | 73 | Floor (mouth) |
| 5 | male | 66 | Lip |
| 6 | male | 54 | Floor (mouth) |
| 7 | female | 59 | Floor (mouth) |
| 8 | male | 72 | Floor (mouth) |
| 9 | male | 60 | Floor (mouth) |
| 10 | male | 59 | Tongue |
| 11 | female | 54 | Floor (mouth) |
| 12 | female | 57 | Bucca |
| 13 | female | 87 | Floor (mouth) |
| 14 | male | 54 | Oropharynx |
| 15 | male | 48 | Floor (mouth) |
| 16 | male | 60 | Tongue |
| 17 | male | 54 | Floor (mouth) |
| 18 | male | 57 | Floor (mouth) |
| 19 | male | 59 | Floor (mouth) |

**Table 2**

| Patient data, Control Group: Patient Information | | | |
|---|---|---|---|
| Patient # | Sex | Age (year) | Tumor localization |
| 1 | female | 65 | Floor (mouth) |
| 2 | male | 52 | Tongue |
| 3 | male | 52 | Tongue |
| 4 | male | 57 | Radix linguae |
| 5 | male | 40 | Tongue |
| 6 | male | 59 | Tongue |
| 7 | male | 43 | Floor (mouth) |
| 8 | Male | 53 | Radix linguae |
| 9 | male | 45 | Tongue |
| 10 | female | 50 | Tongue |
| 11 | male | 66 | Lip |
| 12 | male | 75 | Lip |
| 13 | male | 67 | Lip |
| 14 | male | 55 | Tongue |
| 15 | male | 58 | Radix linguae |
| 16 | female | 53 | Tongue |
| 17 | male | 46 | Tongue |
| 18 | male | 75 | Lip |
| 19 | male | 77 | Lip |
| 20 | male | 61 | Floor (mouth) |

### Inclusion criteria

Twenty-one patients with previously untreated head and neck cancer (Oral Squamous Cell Carcinoma, OSCC) are included in a LI treatment group. The inclusion criteria are 18 years or older having histologically confirmed squamous cell carcinoma of the head and neck with no known metastatic disease and with expected survival of more than 6 months and not treated previously for OSCC or for any other immunotherapy. Patients who are pregnant, had prior radiation to the site of LI administration, presented with duodenal or gastric ulcer, or patients with asthma, are excluded.

### Evaluable Patients

A total of forty-one patients are selected with thirty-nine evaluable. A LI treated group consists of 21 patients with 19 patients evaluable. Two of the LI-treated group patients are subsequently confirmed as having (1) anaplastic carcinoma of the uvula, and (2) adenocarcinoma of the floor of the mouth. These two patients are given the full course of therapy including the administration of the investigational drug but are not part of the histopathology/immunohistochemistry analysis because they did not meet the criteria for having squamous cell carcinoma of the oral cavity. The control group consisted of 20 patients with biopsy confirmed oral squamous cell carcinoma with tumors located in the same areas of the oral cavity and tumor size and disease stage similar to that of the investigational drug (LI) treated group.

### Treatment protocol

Leukocyte Interleukin, Injection (LI) administration is performed in the following manner; one half (400 IU) of the total daily dose (800 IU/mL, as IL-2) is injected peritumorally (1/4 of the peritumoral dose [approximately 100 IU] at each of four sites around the tumor mass) and one half (400 IU, as IL-2), of the total daily dose is administered perilymphatically ipsilateral to the tumor site (sequentially and on the same visit) over a three week period, 5 times per week for three weeks. LI is administered intradermally at the circumferential margins of the visible/palpable tumor mass. The perilymphatic injections are given at the posterior mandibular area in the area of the jugular lymphatic chain ipsilateral to the injected tumor mass.

A single intravenous infusion of cyclophosphamide is given, Inj., 300 mg/m², three days prior to the first LI administration. Indomethacin (25 mg) is self-administered orally (with food), three times daily for a total daily dose of 75 mg beginning 3 days post cyclophosphamide administration and until 24 hours prior to surgery. Zinc Sulfate (50 mg, as elemental Zinc) and multivitamin supplement, once daily, is self-administered beginning 3 days after cyclophosphamide administration and until 24 hours prior to surgery.

All patients (LI-treated and control) receive surgery at 1-2 weeks after the last LI dose followed by radiation therapy at 1-4 weeks after surgery. LI pre-treatment did not adversely affect wound healing in this patient population.

### Patient examination

Prior to the inclusion in the trial patients underwent a general assessment. Their medical history is also reviewed. Once enrolled a complete physical examination, hematological, blood chemistry workup, chest cardioradiography and electrocardiogram were performed. When possible imaging of the primary tumor site is done to obtain a baseline image. All patients have a baseline bi-dimensional measurement of their primary tumor by measuring the two major perpendicular diameters. Patients are interviewed at each subsequent visit and are asked about quality of life (e.g., level of pain, degree of tongue mobility, etc.) using a qualified questionnaire. The treating physician assessed toxicity at each visit.

### Leukocyte Interleukin Injection (LI)

Leukocyte Interleukin Injection (LI) is prepared from selected human peripheral blood mononuclear cells obtained from the US Red Cross after passing all FDA mandated testing for blood for transfusion cultured with mitogen. No first time donors are allowed. The serum free culture supernatant is then aseptically harvested, clarified, subjected to a commercial virus exclusion process, concentrated, and microfiltered. Human Serum Albumin, Inj. USP is added to the concentrate and the resultant solution is buffered to physiological pH, brought to a target IL-2 concentration, and subjected to a second microfiltration. The formulated drug solution is aseptically dispensed into sterile serum-type vials and labeled by its content of IL-2. Product potency is measured by the incorporation of radiolabeled thymidine (in vitro) by the use of a cytotoxic T-lymphoid (CTLL-2), IL-2 dependent cell line. The final injectable agent is further tested by ELISA or the presence of five marker cytokines: IL-2, IL-1β, GM-CSF, IFN-γ, and TNF-α. LI is further subjected to quality control tests for sterility, bacterial endotoxins, pH, and total protein concentration, and other physical-chemical tests.

The Leukocyte Interleukin Injection (LI) is provided frozen in a borosilicate glass serum vial containing 2.2 mL of drug at the label claim as IL-2 (400 IU/ml) for peritumoral, intratumoral, perilymphatic or subcutaneous administration. LI is subjected to quality control tests for identity, sterility, bacterial endotoxins, pH, and total protein concentration. Each vial is inspected for particulate contamination and appearance. The preparation has a total protein content of 3 mg/mL wherein the material is supplied sterile and pyrogen free. LI has an assigned expiration date of 24 months from date of manufacture when the drug is stored at -20°C.

### Cyclophosphamide

Cyclophosphamide, Inj. USP (Bristol-Myers-Squibb, UK) is supplied as a sterile powder containing 45 mg sodium chloride, 75 mg mannitol or approximately 82 mg sodium bicarbonate per 100 mg cyclophosphamide for reconstitution prior to intravenous infusion.

### Indomethacin

Indomethacin USP (Sanofi - Synthelabo, France) is supplied as 25 mg tablets for oral self-administration with food.

### Zinc Sulfate and Multivitamins

Zinc sulfate (50 mg as elemental Zinc, R. P. Scherer Corporation, Clearwater, Florida, USA) and OTC multivitamins are supplied by the clinic to each patient for self-administration.

### Pathology

A single Pathology Protocol describes the preparation and fixation of the surgically excised specimens and the gross, macroscopic and microscopic examination, histology and immunohistochemistry procedures, as described herein. Diagnosis of the oral lesions is determined on excision biopsy of the suspected lesion. The cancer is classified as T2-3N0-2M0. Only T2-3N0-2M0 patients meeting all other inclusion criteria are selected for immunotherapy. Tumor response as determined by bi/tri-dimensional measurements is evaluated at the end of the LI treatment regimen and prior patients scheduled for tumor resection. The excised tissue is placed in pre-labeled containers with saline-buffered formalin, fixed overnight prior to embedding in paraffin and preparing thin section slides for Hematoxylin-Eosin (H&E) staining, immunohistochemistry and pathology evaluation of tumor response.

### Histology

Histopathologic analyses are performed on 3 different tumor-regions: Surface (Zone 1), Center (Zone 2), and Tumor-Stroma interface (Zone 3). Histology and AJCC grading and occurrence of necrotic tumor cells are determined from HE stained sections. The percent of tumor epithelial component versus stroma in the tumors is determined by two methods: (1) connective tissue stained according to Mallory (trichome staining) and (2) the slides being measured for the area of cancer nests (tumor epithelia) by ImagePro analysis software (MediaCibernetics, Silver Spring, MD). Slides containing resected tissue are labeled for cytokeratine using pancytokeratine antibody from DAKO (Glostrup, Denmark) (A1A3+CK19), which labels cancer cells. The slides are examined microscopically and analyzed using ImagePro analysis software.

### Characterization of the mononuclear cell infiltrate

Mononuclear cells present in the close vicinity of tumor cell nests are determined by immunohistochemistry (from paraffin sections). Sections are dewaxed and treated with microwave to retrieve antigenicity. Only commercial antibodies are used, which are previously demonstrated to consistently stain de-waxed paraffin sections. Neutrophils are labeled using anti-myeloperoxidase antibody (mouse monoclonal from DAKO), hematopoietic stem cells are labeled with mouse monoclonal anti-CD34 antibody (DAKO), macrophage cell population is identified by the expression of CD68 antigen (mouse monoclonal anti-CD68, DAKO), dendritic cells are identified by the expression of CD1a marker (mouse monoclonal anti-CD1a, Immunotech, Paris, France). Lymphoid cells are identified by LCA antigen expression (mouse monoclonal anti-CD45, DAKO). T cells are identified by mouse monoclonal anti-CD3 antibody (DAKO). Cytotoxic T cells are identified by the expression of CD8 by anti-CD-8 cytotoxic T cells antibody, DAKO. Effector CD4 T cells are identified using anti-CD4+ T cell antibody (Novocastra, Newcastle Upon Tyne, UK). NK cells are identified using CD56/CD57 antigen mouse monoclonal anti-CD56 and anti-CD57 antibody from Novocastra. In all cases appropriate antibody (same isotype antibody) positive and negative control slides are prepared and evaluated. IL-2R expressing cells, within the tumor epithelia and stroma are identified using a mouse monoclonal antibody to IL-2R, CD25 (Novocastra). In all cases, negative control slides are prepared using isotope-matched monoclonal antibodies non-specific to the target of interest.

All immunohistochemical (IHC) labeling is done with DAKO LSAB-2 kit using a biotinylated anti-mouse/anti-rabbit IgG linker and Streptavidin-HRP to reveal specifically bound antibodies. The chromagen used is AEC (red) label. Sections are counterstained for the nuclei using Hematoxylin.

### Hot spot technique

The density of mononuclear cells is determined based on the "hot spot" technique. Each studied tumor-area density is measured at the region of the highest tumor infiltrating mononuclear cell density. This method minimizes the extreme heterogeneity of the cellular infiltrates in tissues.

### Pathology evaluation

Tumor biopsies from the LI-treated group and control are evaluated with HE staining using microscopic appearance of the tumor. CD3, CD4, CD8, CD1a and CD25 labeling is also performed provided the size of the sample allows for the performance of all 5 labeling procedures. The complete analytical program is employed for the control group and the LI treated group. A 30x magnification is used to select the slides for viewing. A 100x magnification is used during histological analysis. Pathologic and histologic evaluation and morphometric measurements of tissue sections are performed by 3 independent pathologists (JT, CSF and BD) blinded to the study. Other pathologic and histologic evaluation can be used as known within the art.

### Statistical analysis

Pathology data are analyzed by ANOVA, single factor analysis, and *"p" values of* <0.05 (at α=0.05) are considered statistically significant.

### Histological evaluation

The histological diagnosis, Brodes scores and TNM (Tumor Node Metastases) stage of all the LI-treated (19) and control (20) patients are listed in Tables 3 and 4. LI-treated and control did not differ histologically nor with respect to the keratinization (Brodes scores) or TNM stages as shown in Tables 3 and 4 (Odell et al., "The Progostic Value of Individual Histologic Grading Parameters in Small Lingual Squamous Cell Carcinomas; The Importance of the Pattern of Invasion", Cancer 74: 789 (1994)). This is confirmed by the cytokeratin immunostaining patterns showing highly heterogeneous expression of CK-19 and pan-CK in tumors in both groups.

**Table 3**

| Histology, Control Group | | | | |
|---|---|---|---|---|
| Patient # | Dg | Grade | pTNM | Necrosis Type |
| 1 | cc.spinocell. | BR3 | PT2N0 | - |
| 2 | CPC | BR2 | PT2N0 | + macroscopic |
| 3 | CPC | BR3 | PT2N1 | - |
| 4 | CPC | BR3 | PT2N0 | + unicellular |
| 5 | CPC | BR2 | PT2N0 | - |
| 6 | CPC | BR3 | PT2N2 | + macroscopic |
| 7 | CPC | BR2 | PT2N0 | - |
| 8 | CPC | BR3 | PT3N0 | + macroscopic |
| 9 | CPC | BR3 | PT2N0 | - |
| 10 | CPC | BR3 | PT2N0 | - |
| 11 | CPC | BR1 | PT2N0 | + Micro-centr |
| 12 | CPC | BR1 | PT2N0 | - |
| 13 | CPC | BR1 | PT3N0 | - |
| 14 | CPC | BR3 | PT2N0 | - |
| 15 | CPC | BR1 | PT2N0 | + macroscopic |
| 16 | CPC | BR3 | PT3N0 | - |
| 17 | CPC | BR1 | PT2N0 | + centr-micr |
| 18 | CPC | BR2 | PT2N0 | - |
| 19 | CPC | BR1 | PT2N0 | - |
| 20 | CPC | BR1 | PT2N0 | + unicell |

**Table 4**

| Histology and Pathology; Leukocyte Interleukin, Injection Treated Group | | | | | |
|---|---|---|---|---|---|
| Patient # | Dg | Grade | pTNM | Clinical response* | Necrosis Type |
| 1 | CPC | BR3 | T2N0M0 | | Mi, Multifocal |
| 2 | CPC | BR1 | T2N0M0 | | No |
| 3 | CPC basaloid | BR3 | T2N0M0 | | Mi, multifocal |
| 4 | CPC | BR3 | T2N0M0 | MR (40%) | Mi Multifocal |
| 5 | Fibrous stroma | | | CR (100%) | No |
| 6 | CPC | BR3 | T3N0M0 | MR (35%) | No, |
| 7 | CPC | BR2 | T1N0M0 | PR (66%) | Mi Multifocal |
| 8 | CPC | BR2 | T2N0M0 | | No |
| 9 | CPC | BR2 | T2N0M0 | MR (30%) | No, |
| 10 | CPC | BR2 | T2N0M0 | | Mi, Multifocal |
| 11 | CPC | BR2 | T2N0M0 | | Mi, Multifocal |
| 12 | CPC | BR3 | T2N2M0 | MR (35%) | Mi, multifocal |
| 13 | CPC | BR3 | T2N1M0 | | No |
| 14 | dysplasia | | | CR (100%) | No |
| 15 | CPC | BR3 | T2N0M0 | | No |
| 16 | CPC | BR2 | T2N0M0 | | Mi, multifocal |
| 17 | CPC | BR1 | T2N0M0 | | Mi, Necrosis |
| 18 | CPC | BR1 | T2N0M0 | PR (61%) | No |
| 19 | CPC | BR1 | T2N0M0 | | Mi, multifocal |

| | | | | | |
|---|---|---|---|---|---|
| • *As determined by pathology* (Therasse et al. "New Guidelines to Evaluate the Response to Treatment in Solid Tumors", J Nat Can Inst 92 (2000). | | | | | |

Two of the nineteen LI treated patients do not have cancer tissue in the resected tumor. These two patients are considered to be complete clinical responders to LI each exhibiting 100% tumor reduction as shown in Table 4. Two other cases have a histologically verified tumor volume reduction of greater than 50% and are considered to have a partial (major) response. Four additional patients have a volume reduction of greater than 30% but less than 50% and are considered to have a minor response. The objective response rate in the LI-treated group in this trial is 21% with an overall response measured in eight of nineteen patient or 42% as determined by pathology shown in Tables 4 and 5.

**Table 5**

| Pathology grading of cancer (response to treatment) categories* | | |
|---|---|---|
| Grading | Tumor volume/area reduction | Response category |
| I | <30% | No response |
| II | >30 - 49% | Minor response |
| III | >50-99% | Major response (Partial response) |
| IV | 100% | Complete response |

| | | |
|---|---|---|
| ** As determined by pathology* (Therasse et al. "New Guidelines to Evaluate the Response to Treatment in Solid Tumors", J Nat Can Inst 92 (2000). | | |

### Tumor infiltrating lymphocytes

The lymphoid cell infiltrate in the OSCC is predominated by T cells, especially in the tumor stroma. In the control (non LI-treated) group the number of CD8+ T cells that infiltrate the tumor and stroma outnumbered (2:1) the CD4+ T cells as shown in Fig. 1. As a result, the CD4/CD8 ratio in the control (non LI-treated) group is well below 1 (at about 0.5) in all the areas of tumor studied (surface [R1], center [R2], and tumor-stroma interface [R3]) as shown in Fig. 2. LI treatment, on the other hand, induces a significant (p<0.05) increase in the density and number of CD4+ T cells (Fig. 2) and a significant (p<0.05) decrease in the density of CD8+ T cells both in the stroma and the tumor epithelial nests (Fig. 2). This trend is maintained when the same analysis is applied to the treatment responder group alone (Fig.'s 1 and 3). As a consequence, LI treatment caused a marked shift from a low (<1) CD4/CD8 ratio to a high (>2.5 - 3.5) CD4/CD8 ratio in the cellular infiltrate makeup in the OSCC tumor (Fig.'s 2 and 3).

CD34+ mononuclear cells and CD56+ NK cells are not found in the stroma or tumor epithelia nests irrespective of the tumor area or the investigated patient population. Similar findings are reported in LI-treated and control OSCC patients.

The antigen presenting dendritic cells (CD1a+) are almost exclusively located in the tumor epithelial nests and are most abundant at the tumor-stromal interface in the LI-treated group. A differential effect of LI treatment on the distribution of dendritic (CD1a+) cells in the 3 areas investigated (Surface (R1); Tumor Center (R2); and Tumor-Stroma Interface (R3) is observed. In particular, no change is observed in the tumor center; a decrease in CD1a+ dendritic cells is detected at the surface of the tumor and an increase is present at the tumor-stroma interface as shown in Fig. 4. A shift from the surface of the tumor toward the tumor-stroma interface of CD1a+ dendritic cells is more pronounced in the clinical responder subgroup analysis as shown in Fig. 4. The two complete responders have no observable tumor tissue precluding immunohistochemistry analysis.

### Inflammatory cells

The presence of neutrophil and macrophage infiltrate presence in the resected tumor specimens is determined by staining for myeloperoxidase (MPX) and CD68 markers, respectively. Morphometry is performed in the 3 regions of the tumors (surface [R1], center [R2] and tumor stroma interface [R3]).

Analysis of the macrophage-density indicates that LI treatment does not change stromal presence of CD68+ macrophages but decreases significantly (p<0.002) intraepithelially as shown in Fig. 5.

LI treatment significantly (p<0.005) increases the intraepithelial density of neutrophils irrespective of the area (R1, R2 or R3) analysed as shown in Fig. 6.

### Tumor necrosis

Histological appearance of necrosis shows a marked difference between LI-treated and control as shown in Fig. 7. Multifocal microscopic necrosis is markedly more frequent in the LI treated group (10/17 of treated patients) versus the control group (4/20 control patients) as shown in Table 6.

**Table 6**

| Incidence of Necrosis in OSCC tumor samples | | |
|---|---|---|
| Form of Necrosis | CONTROL | Leukocyte Interleukin, Injection |
| No | 12/20 | 7/17 |
| Multifocal/microscopic | 4/20 | 10/17 |
| Macroscopic | 4/20 | 0/17 |

### Tumor stroma / cancer nest ratio

The percentage of cancer cell nest relative to tumor stroma shows the selective effect of LI treatment on cancer nests. The resected tissue samples stained with Mallory trichrome technique having cancer nests labeled pink and the proportion of epithelial nest in the tumor tissue is determined by morphometry using ImagePro software as shown in Fig. 8. LI treatment induces a significant reduction in the proportion of cancer cell nests in tumor tissue (p<0.005, ANOVA single factor analysis) compared to control (non LI-treated).

Periepithelial collagenosis is similar in frequency in both the control and LI-treated patients while interstitial intraepithelial fibrosis is significantly (*p*<0.01) more frequent in the LI-treated group (10/19, or 53% of treated patients) versus the control group (2/20, or 10% of control patients) as shown in Table 7.

**Table 7**

| Incidence of Fibrosis in OSCC tumor samples | | |
|---|---|---|
| Fibrosis | CONTROL | Leukocyte Interleukin, Injection |
| No | 8/20 | 3/19 |
| Periepithelial | 10/20 | 6/19 |
| Stromal | 2/20 | 10/19 |

Leukocyte Interleukin (LI) Injection administered to advanced primary OSCC patients as a first-line treatment prior to conventional therapy induces intra-tumor alterations in infiltrating immune cells rather than inflammatory cells. The T cell population migrates into the cancer nests. The infiltrating CD8+ T cells increases CD25+ expression when LI is administered peritumorally three times a week over a two week period in a range from about 20 IU to 1600 IU or specifically at 400 IU or at 800 IU or still further at five times a week in a range from about 20 IU to 1600 IU or at 400 IU or at 800 IU. Since this treatment regimen does not result in massive necrosis of the tumor tissue at the end of the two-week treatment regimen, there is no marked change in the ratio of connective tissue to the tumor epithelial component.

This is contrasted with the method described herein wherein the method results in a significant reduction in the tumor mass of LI-treated OSCC patients compared to a control group following a three-week pre-treatment with LI prior to surgery followed by radiation therapy. The method contemplates a dose of LI pre-treatment of 800 IU/day as IL-2 for 5 days per week for a total of 3 weeks thereby inducing a significant modulation of the infiltrating immune cells. The number of CD8+ T cells (p<0.05) decrease with a corresponding and significant (p<0.05) increase in tumor infiltrating CD4+ T cells. These changes lead to a fundamental alteration in the CD4/CD8 ratio in the tumor.

The low CD4/CD8 ratio (<1) seen in the control non-treated group of OSCC patients increased dramatically in the stroma and intraepithelially (CD4/CD8 ratio of >2 to >3.5) as a result of LI treatment. Thus, high dose (defined as: 800 IU/day as IL-2, 5 days/week, for 3 weeks) neoadjuvant LI treatment of advanced primary OSCC patients is a paradigm shift of a marked CD4+ T cell infiltrate. A clear and specific change in the density and localization of antigen presenting cells (dendritic cells) and inflammatory cells, especially neutrophils, in the cancer nests is shown by the schematic diagram of host immune activation by LI of Fig. 9.

Complex alterations of the tumor infiltrating cellular component of the host anti-tumor immune response such as multifocal necrosis of cancer nests and an increase in the proportion of connective tissue detected in the LI-treated OSCC patients also occur. These changes reflect the aftermath of a successful anti-tumor immune response raised against OSCC induced by the neoadjuvant LI administration.

A successful immune response to OSCC may require the reversal of the low (<1) intratumoral CD4/CD8 ratio. The predominance of CD8+ T cells in the naïve non-treated OSCC patient over CD4+ T cells within the tumor and the surrounding stroma can be seen in many other solid tumors and is not specific for OSCC. Similarly low CD4/CD8 ratios have been detected in basal cell carcinoma, cervical carcinoma and in breast cancer suggesting an acquired immunosuppressed state of the patient (host) induced by these tumors.

LI-treatment induced histopathological alterations of the cellular infiltrate of the host immune cells in the OSCC results in a 42% overall response rate in the LI pre-treatment group. The objective response rate is 21% with 2 pathology verified complete responses in 19 evaluable LI-treated patients.

Histopathological comparison of the LI-treated group to those described in the LI-treated responder sub-group where clinical and pathology regressions are observed in the eight of nineteen cases reveals that all the studied parameters from immune cell composition to inflammatory cell density and histology of the residual tumor are highly similar in these two sub-groups of LI-treated patients. These data suggest that the LI-treated group responded relatively homogeneously to LI treatment but the resultant anti-cancer pathology effects are different. A plausible explanation for this discrepancy could be the differential immunosensitivity of OSCC patients (differences in Ir-genes and HLA) and/or the immune-competence level (at study entry), which resulted in a gradation of the functional immune response impairment in the different LI-treated patients. Lack of expression either of the MHC-I antigens, co-stimulatory molecules on the target cancer cells and their FAS-Ligand expression, or TCR-ζ chain abnormalities and apoptotic sensitivity of CD8+ T cells and the presence of CD34+ cells can all contribute to the impaired immune response in OSCC (Cruz et al., "Lack of MHC class I surface expression on neoplastic cells and poor activation of the secretory pathway of cytotoxic cells in oral squamous cell carcinomas", Br J Cancer 81:881 (1999); Lang et al., "Impairment of T-cell activation in head and neck cancer in situ and in vitro", Arch Otolaryngol Head Neck Surg 125:82 (1999); Lutz et al., "Human leukocyte antigen class I expression on squamous cell regulates natural killer cell activity", Cancer Res 59:5793 (1999); Hoffmann et al., "Spontaneous apoptosis of circulating T lymphocytes in patients with head and neck cancer and its clinical importance", Clin Cancer Res 8:2553 (2002); Reichert et al., "The number of intratumoral dendritic cells and ξ-chain expression in T cells and prognostic and survival biomarkers in patients with oral carcinoma", Cancer 91:2136 (2001); Kuss et al., "Effector CD8+CD45RO-CD27-T cells have signaling detects patients with squamous cell carcinoma of the head and neck", Br J Cancer 88:223 (2003); Schmidt et al., "Mechanisms of immune suppression in patients with head and neck cancer: presence of CD34+ cells which suppress immune functions within cancers that secrete granulocyte-macrophage colony-stimulating factor", Clin Cancer Res 1:95 (1999); Rivoltini et al., "In vivo interleukin 2-induced activation of lymphokine-activated killer cells and tumor cytotoxic T-cells in cervical lymph nodes of patients with head and neck tumors", Cancer Res 50:5551 (1990); Young et al., "Mechanisms of immune suppression in patients with head and neck cancer: influence on the immune infiltrate of the cancer", Int J Cancer 67:333 (1996). Accordingly, exclusive targeting of the immune system without a concurrent attempt to modulate the sensitivity of the cancer cell target may not result in improvement of the overall response rate to treatment.

LI neoadjuvant pre-treatment of patients with advanced primary OSCC according to the doses described herein results in a paradigm shift in the type of immune cells infiltrating the tumor (predominantly CD4+ T cell rather then CD8+ T cell). This shift results in tumor cell necrosis and a marked reduction in tumor mass compared to a marginal anti-tumor response in the control group having a predominance of CD8+ T cell infiltrate and few tumor infiltrating CD4+ T cells. A paradigm shift in the tumor immune cell infiltrate makeup (a reversal of the intratumoral *CD4*/*CD8* ratio) taken together with the previously reported clinical finding in which LI-treated patients exhibited increased sensitization to radiation therapy of the residual tumor cells, and a biological effect of treatment as measured by the increase time to recurrence and the reduction in the recurrence rate of LI-treated OSCC patients as compared to control (non-treated) OSCC patients results in a new approach to the treatment of OSCC and possibly other solid tumors.

## Claims

1. A therapeutically active amount of a serum-free and mitogen-free cytokine mixture for use in a method for pre-sensitizing cancer by altering the CD4/CD8 ratio in a head and neck tumor, wherein said serum-free and mitogen-free cytokine mixture is administered five times a week over a three week period in an amount of 800 IU wherein IU represent International Units for Interleukin-2 given in World Health Organization 1^{st} International Standard for Human IL-2, 86/504, wherein 400 IU of said 800 IU serum-free and mitogen-free cytokine mixture is injected peritumorally into four different sites around a tumor mass such that 100 IU is injected into each of the four sites and injecting 400 IU/mL perilymphatically ipsilateral to the tumor mass; and wherein
said serum-free and mitogen-free cytokine mixture is comprised of specific ratios of cytokine selected from the group IL-1β, TNF-α, IFN-γ and GM-CSF to Interleukin-2 (IL-2) as follows:
IL-1β to IL-2 at a ratio range of 0.4 - 1.5;
TNF-α to IL-2 at a ratio range of 3.2 - 11.3;
IFN-γ to IL-2 at a ratio range of 1.5 - 10.9; and
GM-CSF to IL-2 at a ratio range of 2.2 - 4.8.

2. The cytokine mixture of claim 1 for the use defined in said claim, wherein said perilymphatic injection of 400 IU is at a posterior mandibular area in the area of the jugular lymphatic chain ipsilateral to the tumor mass.

3. The cytokine mixture of claim 1 or 2 for the use defined in said claim, wherein said specific ratios of cytokines are as follows:
IL-1β to IL-2 at a ratio range of 0.6 to 0.8;
TNF-α to IL-2 at a ratio range of 7.7 to 10.9;
IFN-γ to IL-2 at a ratio range of 4.9 to 7.1; and
GM-CSF to IL-2 at a ratio range of 3.5 to 4.5.

4. A therapeutically active amount of a serum-free and mitogen-free cytokine mixture for use in a method for pre-sensitizing cancer by altering mononuclear cellular infiltrate into a head and neck tumor, wherein said serum-free and mitogen-free cytokine mixture is administered five times a week over a three week period in an amount of 800 IU wherein IU represent International Units for Interleukin-2 given in World Health Organization 1^{st} International Standard for Human IL-2, 85/504, wherein 400 IU of said 800 IU serum-free and mitogen-free cytokine mixture is injected peritumorally into four different sites around a tumor mass such that 100 IU is injected into each of the four sites and injecting 400 IU/mL perilymphatically ipsilateral to the tumor mass; and wherein
said serum-free and mitogen-free cytokine mixture is comprised of specific ratios of cytokines selected from the group of IL-1β+, TNF-α, IFN-γ and GM-CSF to Interleukin-2 (IL-2) as follows:
IL-1β to IL-2 at a ratio range of 0.4 - 1.5;
TNF-α to IL-2 at a ratio range of 3.2 - 11.3;
IFN-γ to IL-2 at a ratio range of 1.5 - 10.9; and
GM-CSF to IL-2 at a ratio range of 2.2 - 4.8

5. The cytokine mixture of claim 4 for the use defined in said claim, wherein said perilymphatic injection of 400 IU is at a posterior mandibular area in the area of the jugular lymphatic chain ipsilateral to the tumor mass.

6. The cytokine mixture of claim 5 for the use defined in said claim, wherein said specific ratios of cytokines are as follows:
IL-1β to IL-2 at a ratio range of 0.6 to 0.8;
TNF-α to IL-2 at a ratio range of 7.7 to 10.9;
IFN-γ to IL-2 at a ratio range of 4.9 to 7.1; and
GM-CSF to IL-2 at a ratio range of 3.5 to 4.5.

7. A cytokine mixture according to any one of the claims 1, 3, 4 and 6 for the use defined in said claims, further comprising an IL-3 to IL-2 ratio in a range from 0.38 - 0.68, preferably at 0.53+/-0.15.

8. A cytokine mixture according to any one of the claims 1, 3, 4 and 6 for the use defined in said claims, further comprising an IL-6 to IL-2 ratio in a range from 37.2 - 53.8, preferably at 46+/-5.9.

9. A cytokine mixture according to any one of the claims 1, 3,4 and 6 for the use defined in said claims, further comprising an IL-8 to IL-2 ratio in a range from 261 - 561.5, preferably at 411+/- 10.6.

10. A cytokine mixture according to any one of the claims 1, 3, 4 and 6 for the use defined in said claims, further comprising an IL-1α to IL-2 ratio in a range from 0.56 - 0.94, preferably at 0.75+/- 0.19.

11. A cytokine mixture according to any one of the claims 1, 3,4 and 6 for the use defined in said claims, further comprising an IL-10 to IL-2 ratio in a range from 2.87 - 3.22, preferably at 3.0+/- 0.18.

12. A cytokine mixture according to any one of the claims 1, 3, 5 and 6 for the use defined in said claims, further comprising an IL-16 to IL-2 ratio in a range from 1.16 - 2.84, preferably at 1.84+/-0.68.

13. A cytokine mixture according to any one of the claims 1, 3, 4 and 6 for the use defined in said claims, further comprising a G-CSF to IL-2 ratio in a range from 2.16 - 3.78, preferably at 2.97+/- 0.81.

14. A cytokine mixture according to any one of the claims 1, 3, 5 and 6 for the use defined in said claims, further comprising a TNF-β to IL-2 ratio in a range from 1.17 - 2.43, preferably at 1.8+/- 0.63.

15. A cytokine mixture according to any one of the claims 1, 3, 4 and 6 for the use defined in said claims, further comprising a MIP-1α to IL-2 ratio in a range from 15.7 - 37.16, preferably at 22.7+/- 7.0.

16. A cytokine mixture according to any one of the claims 1, 3, 4 and 6 for the use defined in said claims, further comprising a MIP-1β to IL-2 ratio in a range from 17.1 - 28.5, preferably at 22.8+/- 5.7.

17. A cytokine mixture according to any one of the claims 1, 3,4 and 6 for the use defined in said claims, further comprising a RANTES to IL-2 ratio in a range from 2.3 - 2.7, preferably at 2.5+/- 0.13.

18. A cytokine mixture according to any one of the claims 1, 3, 4 and 6 for the use defined in said claims, further comprising an EGF to IL-2 ratio in a range from 0.267 - 0.283, preferably at 0.275+/- 0.008.

19. A cytokine mixture according to any one of the claims 1, 3, 5 and 6 for the use defined in said claims, further comprising a PGE₂ to IL-2 ratio in a range from 3.63 - 5.42, preferably at 4.5+/- 0.87.

20. A cytokine mixture according to any one of the claims 1, 3, 4 and 6 for the use defined in said claims, further comprising a TxB₂ to IL-2 ratio in a range from 23.47 - 25.13, preferably at 24.3+/- 0.83.

21. The cytokine mixture of any of the preceding claims for the use defined in said claims, wherein IL-12 is present in only trace quantities.

22. The cytokine mixture of any of the preceding claims for the use defined in said claims, for use in the treatment of a head and neck tumor.

23. The cytokine mixture of claim 22 for the use defined in said claim, wherein the cancer is Oral Squamous Cell Carcinoma (OSCC).

## Patentansprüche

1. Therapeutisch wirksame Menge einer serumfreien und mitogenfreien Zytokinmischung für den Gebrauch in einem Verfahren zum Vorsensibilisieren von Krebs durch Ändern des CD4/CD8-Verhältnisses in einem Kopf-Hals-Tumor, wobei die serumfreie und mitogenfreie Zytokinmischung über einen Zeitraum von drei Wochen in einer Menge von 800 IU fünfmal pro Woche verabreicht wird, wobei IU für internationale Einheiten von Interleukin-2 gemäß dem 1. internationalen Standard für humanes IL-2, 86/504, der Weltgesundheitsorganisation steht, wobei 400 IU der 800 IU serumfreier und mitogenfreier Zytokinmischung peritumoral an vier verschiedenen Stellen um eine Tumormasse herum injiziert werden, sodass jeweils 100 IU in jede der vier Stellen injiziert werden, und 400 IU/mL perilymphatisch auf der Seite der Tumormasse injiziert werden; und wobei die serumfreie und mitogenfreie Zytokinmischung bestimmte Verhältnisse von Zytokinen, die ausgewählt sind aus der Gruppe bestehend aus IL-1β, TNF-α, IFN-γ und GM-CSF, zu Interleukin-2 (IL-2) wie folgt umfasst:
IL-1β zu IL-2 in einem Verhältnisbereich von 0,4-1,5;
TNF-α zu IL-2 in einem Verhältnisbereich von 3,2-11,3;
IFN-γ zu IL-2 in einem Verhältnisbereich von 1,5-10,9; und
GM-CSF zu IL-2 in einem Verhältnisbereich von 2,2-4,8.

2. Zytokinmischung nach Anspruch 1 für den Gebrauch nach diesem Anspruch, wobei die perilymphatische Injektion von 400 IU an einer posterioren mandibularen Stelle des jugularen Lymphstamms auf der Seite der Tumormasse vorgenommen wird.

3. Zytokinmischung nach Anspruch 1 oder 2 für den Gebrauch nach diesem Anspruch, wobei die spezifischen Verhältnisse der Zytokine die Folgenden sind:
IL-1β zu IL-2 in einem Verhältnisbereich von 0,6-0,8;
TNF-α zu IL-2 in einem Verhältnisbereich von 7,7-10,9;
IFN-γ zu IL-2 in einem Verhältnisbereich von 4,9-7,1; und
GM-CSF zu IL-2 in einem Verhältnisbereich von 3,5-4,5.

4. Therapeutisch wirksame Menge einer serumfreien und mitogenfreien Zytokinmischung für den Gebrauch in einem Verfahren zum Vorsensibilisieren von Krebs durch Ändern von mononuklearem zellulären Infiltrat in einen Kopf-Hals-Tumor, wobei die serumfreie und mitogenfreie Zytokinmischung über einen Zeitraum von drei Wochen in einer Menge von 800 IU fünfmal pro Woche verabreicht wird, wobei IU für internationale Einheiten von Interleukin-2 gemäß dem 1. internationalen Standard für humanes IL-2, 85/504, der Weltgesundheitsorganisation steht, wobei 400 IU der 800 IU serumfreier und mitogenfreier Zytokinmischung peritumoral an vier verschiedenen Stellen um eine Tumormasse herum injiziert werden, sodass jeweils 100 IU in jede der vier Stellen injiziert werden, und 400 IU/mL perilymphatisch auf der Seite der Tumormasse injiziert werden; und wobei die serumfreie und mitogenfreie Zytokinmischung bestimmte Verhältnisse von Zytokinen, die ausgewählt sind aus der Gruppe bestehend aus IL-1β, TNF-α, IFN-γ und GM-CSF, zu Interleukin-2 (IL-2) wie folgt umfasst:
IL-1β zu IL-2 in einem Verhältnisbereich von 0,4-1,5;
TNF-α zu IL-2 in einem Verhältnisbereich von 3,2-11,3;
IFN-γ zu IL-2 in einem Verhältnisbereich von 1,5-10,9; und
GM-CSF zu IL-2 in einem Verhältnisbereich von 2,2-4,8.

5. Zytokinmischung nach Anspruch 4 für den Gebrauch nach diesem Anspruch, wobei die perilymphatische Injektion von 400 IU an einer posterioren mandibularen Stelle des jugularen Lymphstamms auf der Seite der Tumormasse vorgenommen wird.

6. Zytokinmischung nach Anspruch 5 für den Gebrauch nach diesem Anspruch, wobei die spezifischen Verhältnisse der Zytokine die Folgenden sind:
IL-1β zu IL-2 in einem Verhältnisbereich von 0,6-0,8;
TNF-α zu IL-2 in einem Verhältnisbereich von 7,7-10,9;
IFN-γ zu IL-2 in einem Verhältnisbereich von 4,9-7,1; und
GM-CSF zu IL-2 in einem Verhältnisbereich von 3,5-4,5.

7. Zytokinmischung nach einem der Ansprüche 1,3,4 und 6 für den Gebrauch nach diesen Ansprüchen, ferner umfassend ein Verhältnis von IL-3 zu IL-2 in einem Bereich von 0,38-0,68, vorzugsweise von 0,53+/-0,15.

8. Zytokinmischung nach einem der Ansprüche 1, 3, 4 und 6 für den Gebrauch nach diesen Ansprüchen, ferner umfassend ein Verhältnis von IL-6 zu IL-2 in einem Bereich von 37,2-53,8, vorzugsweise von 46+/-5,9.

9. Zytokinmischung nach einem der Ansprüche 1, 3, 4 und 6 für den Gebrauch nach diesen Ansprüchen, ferner umfassend ein Verhältnis von IL-8 zu IL-2 in einem Bereich von 261-561,5, vorzugsweise von 411+/-10,6.

10. Zytokinmischung nach einem der Ansprüche 1, 3, 4 und 6 für den Gebrauch nach diesen Ansprüchen, ferner umfassend ein Verhältnis von IL-1α zu IL-2 in einem Bereich von 0,56-0,94, vorzugsweise von 0,75+/-0,19.

11. Zytokinmischung nach einem der Ansprüche 1, 3, 4 und 6 für den Gebrauch nach diesen Ansprüchen, ferner umfassend ein Verhältnis von IL-10 zu IL-2 in einem Bereich von 2,87-3,22, vorzugsweise von 3,0+/-0,18.

12. Zytokinmischung nach einem der Ansprüche 1, 3, 5 und 6 für den Gebrauch nach diesen Ansprüchen, ferner umfassend ein Verhältnis von IL-16 zu IL-2 in einem Bereich von 1,16-2,84, vorzugsweise von 1,84+/-0,68.

13. Zytokinmischung nach einem der Ansprüche 1, 3, 4 und 6 für den Gebrauch nach diesen Ansprüchen, ferner umfassend ein Verhältnis von G-CSF zu IL-2 in einem Bereich von 2,16-3,78, vorzugsweise von 2,97+/-0,81.

14. Zytokinmischung nach einem der Ansprüche 1, 3, 5 und 6 für den Gebrauch nach diesen Ansprüchen, ferner umfassend ein Verhältnis von TNF-β zu IL-2 in einem Bereich von 1,17-2,43, vorzugsweise von 1,8+/-0,63.

15. Zytokinmischung nach einem der Ansprüche 1, 3, 4 und 6 für den Gebrauch nach diesen Ansprüchen, ferner umfassend ein Verhältnis von MIP-1α zu IL-2 in einem Bereich von 15,7-37,16, vorzugsweise von 22,7+/-7,0.

16. Zytokinmischung nach einem der Ansprüche 1, 3, 4 und 6 für den Gebrauch nach diesen Ansprüchen, ferner umfassend ein Verhältnis von MIP-1β zu IL-2 in einem Bereich von 17,1-28,5, vorzugsweise von 22,8+/-5,7.

17. Zytokinmischung nach einem der Ansprüche 1, 3, 4 und 6 für den Gebrauch nach diesen Ansprüchen, ferner umfassend ein Verhältnis von RANTES zu IL-2 in einem Bereich von 2,3-2,7, vorzugsweise von 2,5+/-0,13.

18. Zytokinmischung nach einem der Ansprüche 1, 3, 4 und 6 für den Gebrauch nach diesen Ansprüchen, ferner umfassend ein Verhältnis von EGF zu IL-2 in einem Bereich von 0,267-0,283, vorzugsweise von 0,275+/-0,008.

19. Zytokinmischung nach einem der Ansprüche 1, 3, 5 und 6 für den Gebrauch nach diesen Ansprüchen, ferner umfassend ein Verhältnis von PGE₂ zu IL-2 in einem Bereich von 3,63-5,42, vorzugsweise von 4,5+/-0,87.

20. Zytokinmischung nach einem der Ansprüche 1, 3, 4 und 6 für den Gebrauch nach diesen Ansprüchen, ferner umfassend ein Verhältnis von TxB₂ zu IL-2 in einem Bereich von 23,47-25,13, vorzugsweise von 24,3+/-0,83.

21. Zytokinmischung nach einem der vorhergehenden Ansprüche für den Gebrauch nach diesen Ansprüchen, wobei IL-12 nur in Spurenmengen vorliegt.

22. Zytokinmischung nach einem der vorhergehenden Ansprüche für den Gebrauch nach diesen Ansprüchen, für den Gebrauch beim Behandeln eines Kopf-Hals-Tumors.

23. Zytokinmischung nach Anspruch 22 für den Gebrauch nach diesem Anspruch, wobei der Krebs orales Plattenepithelzellkarzinom (OSCC) ist.

## Revendications

1. Quantité thérapeutiquement active d'un mélange de cytokines exempt de sérum et exempt de mitogène, pour une utilisation dans un procédé de pré-sensibilisation du cancer par altération du rapport CD4/CD8 dans une tumeur de la tête et du cou, dans laquelle ledit mélange de cytokines exempt de sérum et exempt de mitogène est administré cinq fois par semaine sur une période de trois semaines dans une quantité de 800 UI, où UI représente les unités internationales pour l'interleukine-2 indiquées dans la 1^{ère} Norme internationale de l'Organisation mondiale de la Santé relative à l'IL-2 humaine, 86/504, où 400 UI desdites 800 UI de mélange de cytokines exempt de sérum et exempt de mitogène sont injectées par voie péritumorale dans quatre sites différents autour d'une masse tumorale, de sorte que 100 UI sont injectées dans chacun des quatre sites, et d'injection périlymphatique de 400 UI/ml ipsilatéralement à la masse tumorale ; et dans laquelle
ledit mélange de cytokines exempt de sérum et exempt de mitogène est constitué de rapports spécifiques de cytokines choisies dans le groupe constitué par IL-1β, TNF-α, IFN-γ et GM-CSF à l'interleukine-2 (IL-2) de la façon suivante :
IL-1β à IL-2 dans une gamme de rapports de 0,4 à 1,5 ;
TNF-α à IL-2 dans une gamme de rapports de 3,2 à 11,3 ;
IFN-γ à IL-2 dans une gamme de rapports de 1,5 à 10,9 ; et
GM-CSF à IL-2 dans une gamme de rapports de 2,2 à 4,8.

2. Mélange de cytokines selon la revendication 1 pour l'utilisation définie dans ladite revendication, dans lequel ladite injection périlymphatique de 400 UI est effectuée au niveau d'une région mandibulaire postérieure dans la zone de la chaîne lymphatique jugulaire ipsilatéralement à la masse tumorale.

3. Mélange de cytokines selon la revendication 1 ou 2 pour l'utilisation définie dans ladite revendication, dans lequel lesdits rapports spécifiques de cytokines sont les suivants :
IL-1β à IL-2 dans une gamme de rapports de 0,6 à 0,8 ;
TNF-α à IL-2 dans une gamme de rapports de 7,7 à 10,9 ;
IFN-γ à IL-2 dans une gamme de rapports de 4,9 à 7,1 ; et
GM-CSF à IL-2 dans une gamme de rapports de 3,5 à 4,5.

4. Quantité thérapeutiquement active d'un mélange de cytokines exempt de sérum et exempt de mitogène, pour une utilisation dans un procédé de pré-sensibilisation du cancer par altération de l'infiltrat cellulaire mononucléaire dans une tumeur de la tête et du cou, dans laquelle ledit mélange de cytokines exempt de sérum et exempt de mitogène est administré cinq fois par semaine sur une période de trois semaines dans une quantité de 800 UI, où UI représente les unités internationales pour l'interleukine-2 indiquées dans la 1^{ère} Norme internationale de l'Organisation mondiale de la Santé relative à l'IL-2 humaine, 86/504, où 400 UI desdites 800 UI de mélange de cytokines exempt de sérum et exempt de mitogène sont injectées par voie péritumorale dans quatre sites différents autour d'une masse tumorale, de sorte que 100 UI sont injectées dans chacun des quatre sites, et d'injection périlymphatique de 400 UI/ml ipsilatéralement à la masse tumorale ; et dans laquelle
ledit mélange de cytokines exempt de sérum et exempt de mitogène est constitué de rapports spécifiques de cytokines choisies dans le groupe constitué par IL-1β, TNF-α, IFN-γ et GM-CSF à l'interleukine-2 (IL-2) de la façon suivante :
IL-1β à IL-2 dans une gamme de rapports de 0,4 à 1,5 ;
TNF-α à IL-2 dans une gamme de rapports de 3,2 à 11,3 ;
IFN-γ à IL-2 dans une gamme de rapports de 1,5 à 10,9 ; et
GM-CSF à IL-2 dans une gamme de rapports de 2,2 à 4,8.

5. Mélange de cytokines selon la revendication 4 pour l'utilisation définie dans ladite revendication, dans lequel ladite injection périlymphatique de 400 UI est effectuée au niveau d'une région mandibulaire postérieure dans la zone de la chaîne lymphatique jugulaire ipsilatéralement à la masse tumorale.

6. Mélange de cytokines selon la revendication 5 pour l'utilisation définie dans ladite revendication, dans lequel lesdits rapports spécifiques de cytokines sont les suivants :
IL-1β à IL-2 dans une gamme de rapports de 0,6 à 0,8 ;
TNF-α à IL-2 dans une gamme de rapports de 7,7 à 10,9 ;
IFN-γ à IL-2 dans une gamme de rapports de 4,9 à 7,1 ; et
GM-CSF à IL-2 dans une gamme de rapports de 3,5 à 4,5.

7. Mélange de cytokines selon l'une quelconque des revendications 1, 3, 4 et 6 pour l'utilisation définie dans lesdites revendications, comprenant en outre un rapport de l'IL-3 à l'IL-2 dans une gamme de 0,38 à 0,68, de préférence de 0,53 ± 0,15.

8. Mélange de cytokines selon l'une quelconque des revendications 1, 3, 4 et 6 pour l'utilisation définie dans lesdites revendications, comprenant en outre un rapport de l'IL-6 à l'IL-2 dans une gamme de 37,2 à 53,8, de préférence de 46 ± 5,9.

9. Mélange de cytokines selon l'une quelconque des revendications 1, 3, 4 et 6 pour l'utilisation définie dans lesdites revendications, comprenant en outre un rapport de l'IL-8 à l'IL-2 dans une gamme de 261 à 561,5, de préférence de 411 ± 10,6.

10. Mélange de cytokines selon l'une quelconque des revendications 1, 3, 4 et 6 pour l'utilisation définie dans lesdites revendications, comprenant en outre un rapport de l'IL-1α à l'IL-2 dans une gamme de 0,56 à 0,94, de préférence de 0,75 ± 0,19.

11. Mélange de cytokines selon l'une quelconque des revendications 1, 3, 4 et 6 pour l'utilisation définie dans lesdites revendications, comprenant en outre un rapport de l'IL-10 à l'IL-2 dans une gamme de 2,87 à 3,22, de préférence de 3,0 ± 0,18.

12. Mélange de cytokines selon l'une quelconque des revendications 1, 3, 5 et 6 pour l'utilisation définie dans lesdites revendications, comprenant en outre un rapport de l'IL-16 à l'IL-2 dans une gamme de 1,16 à 2,84, de préférence de 1,84 ± 0,68.

13. Mélange de cytokines selon l'une quelconque des revendications 1, 3, 4 et 6 pour l'utilisation définie dans lesdites revendications, comprenant en outre un rapport du GM-CSF à l'IL-2 dans une gamme de 2,16 à 3,78, de préférence de 2,97 ± 0,81.

14. Mélange de cytokines selon l'une quelconque des revendications 1, 3, 5 et 6 pour l'utilisation définie dans lesdites revendications, comprenant en outre un rapport du TNF-β à l'IL-2 dans une gamme de 1,17 à 2,43, de préférence de 1,8 ± 0,63.

15. Mélange de cytokines selon l'une quelconque des revendications 1, 3, 4 et 6 pour l'utilisation définie dans lesdites revendications, comprenant en outre un rapport de la MIP-1α à l'IL-2 dans une gamme de 15,7 à 37,16, de préférence de 22,7 ± 7,0.

16. Mélange de cytokines selon l'une quelconque des revendications 1, 3, 4 et 6 pour l'utilisation définie dans lesdites revendications, comprenant en outre un rapport de la MIP-1β à l'IL-2 dans une gamme de 17,1 à 28,5, de préférence de 22,8 ± 5,7.

17. Mélange de cytokines selon l'une quelconque des revendications 1, 3, 4 et 6 pour l'utilisation définie dans lesdites revendications, comprenant en outre un rapport de la RANTES à l'IL-2 dans une gamme de 2,3 à 2,7, de préférence de 2,5 ± 0,13.

18. Mélange de cytokines selon l'une quelconque des revendications 1, 3, 4 et 6 pour l'utilisation définie dans lesdites revendications, comprenant en outre un rapport de l'EGF à l'IL-2 dans une gamme de 0,267 à 0,283, de préférence de 0,275 ± 0,008.

19. Mélange de cytokines selon l'une quelconque des revendications 1, 3, 5 et 6 pour l'utilisation définie dans lesdites revendications, comprenant en outre un rapport de la PGE₂ à l'IL-2 dans une gamme de 3,63 à 5,42, de préférence de 4,5 ± 0,87.

20. Mélange de cytokines selon l'une quelconque des revendications 1, 3, 4 et 6 pour l'utilisation définie dans lesdites revendications, comprenant en outre un rapport du TxB₂ à l'IL-2 dans une gamme de 23,47 à 25,13, de préférence de 24,3 ± 0,83.

21. Mélange de cytokines selon l'une quelconque des revendications précédentes pour l'utilisation définie dans lesdites revendications, où l'IL-2 est seulement présente en quantité infime.

22. Mélange de cytokines selon l'une quelconque des revendications précédentes pour l'utilisation définie dans lesdites revendications, pour une utilisation dans le traitement d'une tumeur de la tête et du cou.

23. Mélange de cytokines selon la revendication 22 pour l'utilisation définie dans ladite revendication, dans lequel le cancer est le carcinome épidermoïde de la cavité buccale (CECB).
